# EUROPEAN PATENT APPLICATION

(11) **EP 4 328 228 A1**
(43) Date of publication of application: **28.02.2024**
(21) Application number: 22806337.6
(22) Date of filing: 30.03.2022
(51) Int. Cl.: C07D 491/107, A61K 31/497, A61K 31/506, A61P 35/00

(54) **HETEROCYCLIC COMPOUND FOR INHIBITING SHP2 ACTIVITY, PREPARATION METHOD THEREFOR AND USE THEREOF**

(30) Priority: 13.05.2021 CN 202110523423
(71) Applicant: Shanghai Institute of Materia Medica, Chinese Academy of Sciences, Pudong, Shanghai 201203 (CN)
(72) Inventor: ZHOU, Bing, Shanghai 201203 (CN); LI, Jia, Shanghai 201203 (CN); ZHOU, Yubo, Shanghai 201203 (CN); YANG, Xiangbo, Shanghai 201203 (CN); FENG, Bo, Shanghai 201203 (CN); YANG, Yaxi, Shanghai 201203 (CN); HU, Xiaobei, Shanghai 201203 (CN)
(74) Representative: Robba, Pierpaolo
(86) International application number: PCT/CN2022/084047
(87) International publication number: WO 2022/237367

(57) **Abstract**

The present invention relates to a heterocyclic compound for inhibiting SHP2 activity that is represented by formula I, a preparation method therefor and use thereof. The compound of the present invention is effective in treating a disease or disorder or condition mediated by SHP2.

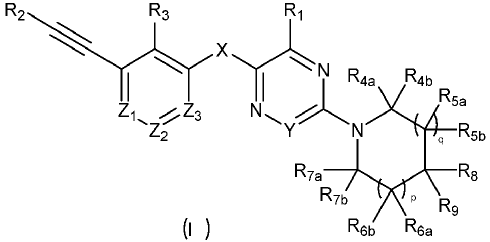

## Description

### Technical field

The present invention relates to compounds capable of inhibiting SHP2 activity. Specifically, the present invention relates to a heterocyclic compound that inhibits SHP2 activity, the preparation method therefor, a pharmaceutical composition comprising the compound, method for treating a SHP2-mediated disease or disorder with the compound or composition, and use of the compound or composition in preparing a medicament.

### Background Art

SHP2 (Src-homology containing phosphatase 2) is a non-receptor protein tyrosine phospholipase encoded by PTPN11. SHP2 structurally contains two Src homology-2 domains (i.e., N-SH2 and C-SH2), a catalytic domain (PTP), and a C-terminal tail. In the resting state, the interaction between the amino acid residues on the N-SH2 domain and the PTP domain maintains the SHP2 molecule in a non-activated autoinhibited conformation; under the stimulation of cytokines, growth factors, etc., SHP2 exposes the catalytic site, resulting in catalytic activity.

SHP2 is widely expressed in various tissue cells, and participates in the process of intracellular signal transduction through Ras-MAPK, JAK-STAT, PI3K-AKT and other signaling pathways, and plays an important role in various cell functions such as cell proliferation, differentiation, migration and cycle maintenance.

High activation of SHP2 catalytic function caused by germline or somatic mutations of PTPN11 has been confirmed in patients with congenital diseases, such as Noonan syndrome, LEOPARD syndrome, and hematological tumors, such as juvenile myelomonocytic leukemia and myelodysplastic syndrome, B-cell acute lymphoblastic leukemia/lymphoma, acute myeloid leukemia, etc.. In addition, activating mutations of PTPN11 also exist in a variety of solid tumors such as lung cancer, colon cancer, melanoma, neuroblastoma, and liver cancer. Meanwhile, SHP2 also plays an important role in PD-1/PD-L1-mediated tumor immune evasion.

Therefore, the activation or upregulation of SHP2 protein in human tumors and other diseases makes it an ideal target for the development of innovative therapies. The compounds of the present invention satisfy the need for small molecules to inhibit SHP2 activity.

### Summary of the Invention

It is an object of the present invention to provide a heterocyclic compound capable of inhibiting SHP2 activity.

It is another object of the present invention to provide a pharmaceutical composition comprising said heterocyclic compound.

Another object of the present invention is to provide a method for treating a disease or disorder associated with abnormal SHP2 activity with the compound or composition.

It is yet another object of the present invention to provide the use of the compound or the composition in the preparation of a medicament for treating diseases or disorders associated with abnormal SHP2 activity.

According to one aspect of the present invention, it provides a compound represented by formula I, or a pharmaceutically acceptable salt, enantiomer, diastereomer, atropisomer, racemate, polymorph, solvate or isotopically labeled compound thereof: 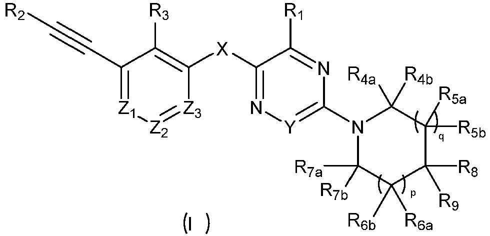 wherein
R₁ is selected from hydrogen, -NH₂, -OH, -CN, halogen, C₁-C₄ alkyl, C₁-C₄ haloalkyl, and C₃-C₆ cycloalkyl;
R₂ is selected from hydrogen, C₁-C₁₀ alkyl, C₁-C₁₀ haloalkyl, C₃-C₈ cycloalkyl, 3- to 10-membered heterocyclyl, C₆-C₁₀ aryl, and 5- to 10-membered heteroaryl; the C₁-C₁₀ alkyl, C₁-C₁₀ haloalkyl, C₃-C₈ cycloalkyl, 3- to 10-membered heterocycloalkyl, C₆-C₁₀ aryl, and 5- to 10-membered heteroaryl are optionally further substituted by one to five substituents selected from -NR₁₀R₁₁, -OR₁₀, -CN, halogen, C₁-C₁₀ alkyl, C₁-C₁₀ haloalkyl, C₃-C₈ cycloalkyl, 3- to 10-membered heterocyclyl, -C(=O)R₁₀, -C(=O)OR₁₀, -C(=O)NR₁₀R₁₁, -S(=O)₂R₁₀, -S(=O)₂NR₁₀R₁₁, and -NR₁₀C(=O)R₁₁; wherein, R₁₀ and R₁₁ are independently selected from hydrogen, C₁-C₄ alkyl, and C₃-C₇ cycloalkyl;
R₃ is selected from hydrogen, halogen, C₁-C₄ alkyl, C₃-C₈ cycloalkyl, C₁-C₄ alkoxy, and C₁-C₄ haloalkyl;
R₄ₐ, R_{4b}, R₅ₐ, R_{5b}, R₆ₐ, R_{6b}, R₇ₐ and R_{7b} are each independently selected from hydrogen, -NH₂, -OH, halogen, carbonyl, C₁-C₄ alkyl, C₁-C₄ alkoxy, C₃-C₈ cycloalkyl and C₁-C₄ alkylamino;
R₈ and R₉ are each independently selected from -NH₂, -OH, -CN, -COOH, halogen, C₁-C₄ alkyl, C₁-C₄ alkoxy, and C₁-C₄ alkylamino; wherein the C₁-C₄ alkyl is optionally substituted by one to three substituents selected from -NH₂, -OH, and -CN;
alternatively, any two groups selected from R₄ₐ, R_{4b}, R₅ₐ, R_{5b}, R₆ₐ, R_{6b}, R₇ₐ, R_{7b} and R₈ are combined with the carbon atoms connected thereto to form a 5- to 6-membered saturated or unsaturated ring;
alternatively, R₈ and R₉ form a 3- to 7-membered saturated or unsaturated ring together with the carbon atom connected thereto, and the 3- to 7-membered saturated or unsaturated ring optionally contains one to three heteroatoms or groups independently selected from N, O, C(=O), and S(=O)ₘ, wherein m=1 or 2; the saturated or unsaturated ring is optionally substituted by one to three substituents selected from -NH₂, -OH, -CN, halogen, C₁-C₄ alkyl, C₁-C₄ alkoxy, and C₁-C₄ alkylamino;
p is 0 or 1;
q is 0 or 1;
X is S or absent;
Yis CR₁₂ or N;
R₁₂ is selected from hydrogen, -NH₂, -CN, halogen, C₁-C₄ alkyl, -OR₁₃, C₃-C₈ cycloalkyl, 3- to 10-membered heterocyclyl, -C(=O)R₁₃, -C(=O)OR₁₃, -C(=O)NR₁₃R₁₄, -S(=O)₂NR₁₃R₁₄ and -S(=O)₂R₁₃, wherein R₁₃ and R₁₄ are each independently selected from hydrogen, C₁-C₄ alkyl and C₃-C₆ cycloalkyl;
Z₁ is N or CR₁₅;
Z₂ is N or CR₁₆;
Z₃ is N or CR₁₇;
R₁₅, R₁₆, R₁₇ are each independently selected from hydrogen, -CN, halogen, -C(=O)R₁₈, -C(=O)OR₁₈, -C(=O)NR₁₈R₁₉, -NR₁₈R₁₉, -NR₁₈C(=O)R₁₉, -NR₁₈C(=O)OR₁₉, -OC(=O)R₁₈, -OC(=O)NR₁₈R₁₉, -OR₁₈, -S(=O)₂NR₁₈R₁₉, -S(=O)₂R₁₈, C₁-C₄ alkyl, C₃-C₈ cycloalkyl, 3- to 10-membered heterocyclyl, C₆-C₁₀ aryl and 5- to 10-membered heteroaryl; R₁₈ and R₁₉ are each independently selected from hydrogen, C₁-C₄ alkyl and C₃-C₈ cycloalkyl; the C₁-C₄ alkyl, C₃-C₈ cycloalkyl in R₁₅ to R₁₉, the 3- to 10-membered heterocyclyl, C₆-C₁₀ aryl, 5- to 10-membered heteroaryl in R₁₅ to R₁₇ are optionally further substituted by one to three substituents independently selected from -NH₂, -OH, -CN, halogen, C₁-C₄ alkyl, C₃-C₆ cycloalkyl and C₁-C₄ alkoxy.

According to another aspect of the present invention, it provides a pharmaceutical composition comprising a therapeutically effective amount of one or more selected from the above compound, and the pharmaceutically acceptable salt, enantiomer, diastereomer, atropisomer, racemate, polymorph, solvate and isotopically labeled compound thereof, and a pharmaceutically acceptable carrier.

According to another aspect of the present invention, it provides a method of treating a disease or disorder or condition mediated by SHP2 with the compound, or the pharmaceutically acceptable salt, enantiomer, diastereomer, atropisomer, racemate, polymorph, solvate or isotopically labeled compound thereof, or the pharmaceutical composition.

According to another aspect of the present invention, it provides the use of the compound or the pharmaceutically acceptable salt, enantiomer, diastereomer, atropisomer, racemate, polymorph, solvate or isotopically labeled compound thereof, or the pharmaceutical composition for preparing a medicament for treating a disease or disorder or condition mediated by SHP2.

### Beneficial effect

The compounds of the present invention are effective in treating a disease or disorder or condition mediated by SHP2.

### Detailed Embodiments

According to an embodiment of the present invention, it provides a compound represented by formula I, or a pharmaceutically acceptable salt, enantiomer, diastereomer, atropisomer, racemate, polymorph, solvate or isotopically labeled compound thereof: 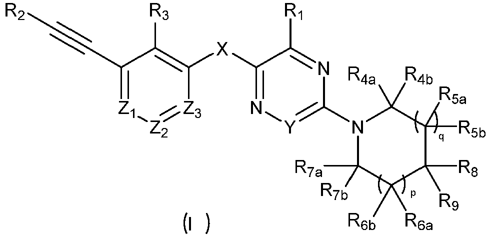 wherein
R₁ is selected from hydrogen, -NH₂, -OH, -CN, halogen, C₁-C₄ alkyl, C₁-C₄ haloalkyl, and C₃-C₆ cycloalkyl;
R₂ is selected from hydrogen, C₁-C₁₀ alkyl, C₁-C₁₀ haloalkyl, C₃-C₈ cycloalkyl, 3- to 10-membered heterocyclyl, C₆-C₁₀ aryl, and 5- to 10-membered heteroaryl; the C₁-C₁₀ alkyl, C₁-C₁₀ haloalkyl, C₃-C₈ cycloalkyl, 3- to 10-membered heterocycloalkyl, C₆-C₁₀ aryl, and 5- to 10-membered heteroaryl are optionally further substituted by one to five substituents selected from -NR₁₀R₁₁, -OR₁₀, -CN, halogen, C₁-C₁₀ alkyl, C₁-C₁₀ haloalkyl, C₃-C₈ cycloalkyl, 3- to 10-membered heterocyclyl, -C(=O)R₁₀, -C(=O)OR₁₀, -C(=O)NR₁₀R₁₁, -S(=O)₂R₁₀, -S(=O)₂NR₁₀R₁₁, and -NR₁₀C(=O)R₁₁; wherein, R₁₀ and R₁₁ are independently selected from hydrogen, C₁-C₄ alkyl, and C₃-C₇ cycloalkyl;
R₃ is selected from hydrogen, halogen, C₁-C₄ alkyl, C₃-C₈ cycloalkyl, C₁-C₄ alkoxy, and C₁-C₄ haloalkyl;
R₄ₐ, R_{4b}, R₅ₐ, R_{5b}, R₆ₐ, R_{6b}, R₇ₐ and R_{7b} are each independently selected from hydrogen, -NH₂, -OH, halogen, carbonyl, C₁-C₄ alkyl, C₁-C₄ alkoxy, C₃-C₈ cycloalkyl and C₁-C₄ alkylamino;
R₈ and R₉ are each independently selected from -NH₂, -OH, -CN, -COOH, halogen, C₁-C₄ alkyl, C₁-C₄ alkoxy, and C₁-C₄ alkylamino; wherein the C₁-C₄ alkyl is optionally substituted by one to three substituents selected from -NH₂, -OH, and -CN;
alternatively, any two groups selected from R₄ₐ, R_{4b}, R₅ₐ, R_{5b}, R₆ₐ, R_{6b}, R₇ₐ, R_{7b} and R₈ are combined with the carbon atoms connected thereto to form a 5- to 6-membered saturated or unsaturated ring;
alternatively, R₈ and R₉ form a 3- to 7-membered saturated or unsaturated ring together with the carbon atom connected thereto, and the 3- to 7-membered saturated or unsaturated ring optionally contains one to three heteroatoms or groups independently selected from N, O, C(=O), and S(=O)ₘ, wherein m=1 or 2; the saturated or unsaturated ring is optionally substituted by one to three substituents selected from -NH₂, -OH, -CN, halogen, C₁-C₄ alkyl, C₁-C₄ alkoxy, and C₁-C₄ alkylamino;
p is 0 or 1;
q is 0 or 1;
X is S or absent;
Yis CR₁₂ or N;
R₁₂ is selected from hydrogen, -NH₂, -CN, halogen, C₁-C₄ alkyl, -OR₁₃, C₃-C₈ cycloalkyl, 3- to 10-membered heterocyclyl, -C(=O)R₁₃, -C(=O)OR₁₃, -C(=O)NR₁₃R₁₄, -S(=O)₂NR₁₃R₁₄ and -S(=O)₂R₁₃, wherein R₁₃ and R₁₄ are each independently selected from hydrogen, C₁-C₄ alkyl and C₃-C₆ cycloalkyl;
Z₁ is N or CR₁₅;
Z₂ is N or CR₁₆;
Z₃ is N or CR₁₇;
R₁₅, R₁₆, R₁₇ are each independently selected from hydrogen, -CN, halogen, -C(=O)R₁₈, -C(=O)OR₁₈, -C(=O)NR₁₈R₁₉, -NR₁₈R₁₉, -NR₁₈C(=O)R₁₉, -NR₁₈C(=O)OR₁₉, -OC(=O)R₁₈, -OC(=O)NR₁₈R₁₉, -OR₁₈, -S(=O)₂NR₁₈R₁₉, -S(=O)₂R₁₈, C₁-C₄ alkyl, C₃-C₈ cycloalkyl, 3- to 10-membered heterocyclyl, C₆-C₁₀ aryl and 5- to 10-membered heteroaryl; R₁₈ and R₁₉ are each independently selected from hydrogen, C₁-C₄ alkyl and C₃-C₈ cycloalkyl; the C₁-C₄ alkyl, C₃-C₈ cycloalkyl in R₁₅ to R₁₉, the 3- to 10-membered heterocyclyl, C₆-C₁₀ aryl, 5- to 10-membered heteroaryl in R₁₅ to R₁₇ are optionally further substituted by one to three substituents independently selected from -NH₂, -OH, -CN, halogen, C₁-C₄ alkyl, C₃-C₆ cycloalkyl and C₁-C₄ alkoxy.

According to an embodiment of the present invention, the compound represented by formula I is selected from the compounds represented by formula Ia: 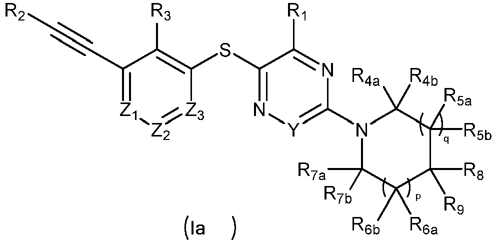 wherein
R₁ is selected from hydrogen, -NH₂, C₁-C₄ alkyl and C₁-C₄ haloalkyl;
R₂ is selected from hydrogen, C₁-C₁₀ alkyl, C₁-C₁₀ haloalkyl, C₃-C₈ cycloalkyl, 3- to 10-membered heterocyclyl, C₆-C₁₀ aryl, and 5- to 10-membered heteroaryl; the C₁-C₁₀ alkyl, C₁-C₁₀ haloalkyl, C₃-C₈ cycloalkyl, 3- to 10-membered heterocycloalkyl, C₆-C₁₀ aryl, and 5- to 10-membered heteroaryl are optionally further substituted by one to five substituents selected from -NR₁₀R₁₁, -OR₁₀, -CN, halogen, C₁-C₁₀ alkyl, C₁-C₁₀ haloalkyl, C₃-C₈ cycloalkyl, 3- to 10-membered heterocyclyl, -C(=O)R₁₀, -C(=O)OR₁₀, -C(=O)NR₁₀R₁₁, -S(=O)₂R₁₀, -S(=O)₂NR₁₀R₁₁, and -NR₁₀C(=O)R₁₁; wherein, R₁₀ and R₁₁ are independently selected from hydrogen, C₁-C₄ alkyl, and C₃-C₇ cycloalkyl;
R₃ is selected from hydrogen, C₁-C₄ alkyl and halogen;
R₄ₐ, R_{4b}, R₅ₐ, R_{5b}, R₆ₐ, R_{6b}, R₇ₐ and R_{7b} are each independently selected from hydrogen, -NH₂, -OH, halogen, carbonyl, C₁-C₄ alkyl, C₁-C₄ alkoxy, C₃-C₈ cycloalkyl and C₁-C₄ alkylamino;
R₈ and R₉ are each independently selected from -NH₂, -OH, -CN, -COOH, halogen, C₁-C₄ alkyl, C₁-C₄ alkoxy, and C₁-C₄ alkylamino; wherein the C₁-C₄ alkyl is optionally substituted by one to three substituents selected from -NH₂, -OH, and -CN;
alternatively, R₈ and R₉ form a 3- to 7-membered saturated or unsaturated ring together with the carbon atom connected thereto, and the 3- to 7-membered saturated or unsaturated ring optionally contains one to three heteroatoms or groups independently selected from N, O, C(=O), and S(=O)ₘ, wherein m=1 or 2; the saturated or unsaturated ring is optionally substituted by one to three substituents selected from -NH₂, -OH, -CN, halogen, C₁-C₄ alkyl, C₁-C₄ alkoxy, and C₁-C₄ alkylamino;
p is 0 or 1;
q is 0 or 1;
Yis CR₁₂ or N;
R₁₂ is selected from hydrogen, -NH₂, -CN, halogen, C₁-C₄ alkyl, -OR₁₃, C₃-C₈ cycloalkyl, 3- to 10-membered heterocyclyl, -C(=O)R₁₃, -C(=O)OR₁₃, -C(=O)NR₁₃R₁₄, -S(=O)₂NR₁₃R₁₄ and -S(=O)₂R₁₃, wherein R₁₃ and R₁₄ are each independently selected from hydrogen, C₁-C₄ alkyl and C₃-C₆ cycloalkyl;
Z₁ is N or CR₁₅;
Z₂ is N or CR₁₆;
Z₃ is N or CR₁₇;
R₁₅, R₁₆, R₁₇ are each independently selected from hydrogen, -CN, halogen, C₁-C₄ alkyl, C₃-C₈ cycloalkyl, 3- to 10-membered heterocyclyl, C₆-C₁₀ aryl and 5- to 10-membered heteroaryl; the C₁-C₄ alkyl, C₃-C₈ cycloalkyl, 3- to 10-membered heterocyclyl, C₆-C₁₀ aryl, 5-to 10-membered heteroaryl are optionally further substituted by one to three substituents independently selected from -NH₂, -OH, -CN, halogen, C₁-C₄ alkyl, C₃-C₆ cycloalkyl and C₁-C₄ alkoxy.

According to an embodiment of the present invention, the compound represented by formula I is selected from the compounds represented by formula Ib: 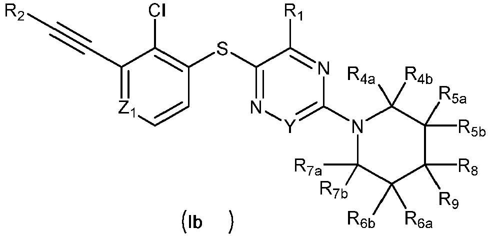 wherein
R₁ is selected from hydrogen, -NH₂, methyl and trifluoromethyl;
R₂ is selected from hydrogen, C₁-C₁₀ alkyl, C₁-C₁₀ haloalkyl, C₃-C₈ cycloalkyl, 3- to 10-membered heterocyclyl, C₆-C₁₀ aryl, and 5- to 10-membered heteroaryl; the C₁-C₁₀ alkyl, C₁-C₁₀ haloalkyl, C₃-C₈ cycloalkyl, 3- to 10-membered heterocycloalkyl, C₆-C₁₀ aryl, and 5- to 10-membered heteroaryl are optionally further substituted by one to five substituents selected from -NR₁₀R₁₁, -OR₁₀, -CN, halogen, C₁-C₁₀ alkyl, C₁-C₁₀ haloalkyl, C₃-C₈ cycloalkyl, 3- to 10-membered heterocyclyl, -C(=O)R₁₀, -C(=O)OR₁₀, -C(=O)NR₁₀R₁₁, -S(=O)₂R₁₀, -S(=O)₂NR₁₀R₁₁, and -NR₁₀C(=O)R₁₁; wherein, R₁₀ and R₁₁ are independently selected from hydrogen and C₁-C₄ alkyl;
R₄ₐ, R_{4b}, R₅ₐ, R_{5b}, R₆ₐ, R_{6b}, R₇ₐ and R_{7b} are each independently selected from hydrogen, -NH₂, -OH, halogen, carbonyl, C₁-C₄ alkyl, C₁-C₄ alkoxy, C₃-C₈ cycloalkyl and C₁-C₄ alkylamino;
R₈ and R₉ are each independently selected from -NH₂, -OH, -CN, -COOH, halogen, C₁-C₄ alkyl, C₁-C₄ alkoxy, and C₁-C₄ alkylamino; wherein the C₁-C₄ alkyl is optionally substituted by one to three substituents selected from -NH₂, -OH, and -CN;
alternatively, R₈ and R₉ form a 3- to 7-membered saturated or unsaturated ring together with the carbon atom connected thereto, and the 3- to 7-membered saturated or unsaturated ring optionally contains one to three heteroatoms or groups independently selected from N, O, C(=O), and S(=O)ₘ, wherein m=1 or 2; the saturated or unsaturated ring is optionally substituted by one to three substituents selected from -NH₂, -OH, -CN, halogen, C₁-C₄ alkyl, C₁-C₄ alkoxy, and C₁-C₄ alkylamino;
Y is CR₁₂ or N;
R₁₂ is selected from hydrogen, -CN and halogen;
Z₁ is N or CR₁₅;
R₁₅ is selected from hydrogen, -CN, halogen, C₁-C₄ alkyl, C₃-C₈ cycloalkyl, 3- to 10-membered heterocyclyl, C₆-C₁₀ aryl and 5- to 10-membered heteroaryl; the C₁-C₄ alkyl, C₃-C₈ cycloalkyl, 3- to 10-membered heterocyclyl, C₆-C₁₀ aryl, 5- to 10-membered heteroaryl are optionally further substituted by one to three substituents independently selected from -NH₂, -OH, -CN, halogen, C₁-C₄ alkyl, C₃-C₆ cycloalkyl and C₁-C₄ alkoxy.

According to an embodiment of the present invention, the compound represented by formula I is selected from the compounds represented by formula Ic: 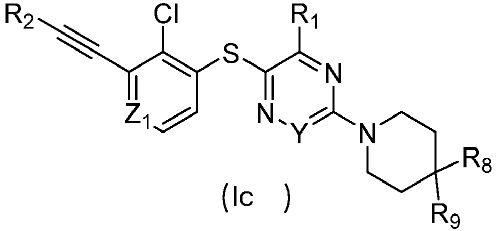 wherein
R₁ is selected from hydrogen and -NH₂;
R₂ is selected from hydrogen, C₁-C₁₀ alkyl, C₃-C₈ cycloalkyl, 3- to 10-membered heterocyclyl, C₆-C₁₀ aryl, and 5- to 10-membered heteroaryl; the C₁-C₁₀ alkyl, C₃-C₈ cycloalkyl, 3- to 10-membered heterocycloalkyl, C₆-C₁₀ aryl, and 5- to 10-membered heteroaryl are optionally further substituted by one to five substituents selected from -NR₁₀R₁₁, -OR₁₀, -CN, halogen, C₁-C₃ alkyl, C₃-C₆ cycloalkyl, -C(=O)R₁₀, -C(=O)OR₁₀, -C(=O)NR₁₀R₁₁, -S(=O)₂R₁₀, -S(=O)₂NR₁₀R₁₁, and -NR₁₀C(=O)R₁₁; wherein, R₁₀ and R₁₁ are independently selected from hydrogen and C₁-C₄ alkyl;
R₈ and R₉ are each independently selected from -NH₂, -OH, -CN, -COOH, halogen, C₁-C₄ alkyl, C₁-C₄ alkoxy, and C₁-C₄ alkylamino; wherein the C₁-C₄ alkyl is optionally substituted by one to three substituents selected from -NH₂ and -OH;
alternatively, R₈ and R₉ form a 3- to 7-membered saturated or unsaturated ring with the carbon atom connected thereto, and the 3- to 7-membered saturated or unsaturated ring optionally contains one to three heteroatoms or groups independently selected from N, O, C(=O), and S(=O)ₘ, wherein m=1 or 2; the saturated or unsaturated ring is optionally substituted by one to three substituents selected from -NH₂, -OH, C₁-C₄ alkyl, C₁-C₄ alkoxy, C₁-C₄ alkylamino and halogen;
Y is CH or N;
Z₁ is N or CR₁₅;
R₁₅ is selected from hydrogen, -CN, C₁-C₄ alkyl, C₃-C₈ cycloalkyl and 3- to 10-membered heterocyclyl.

According to an embodiment of the present invention, the compound represented by formula I is selected from the compounds represented by formula Id: 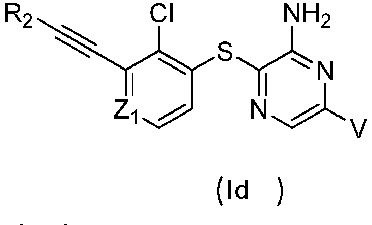 wherein
R₂ is selected from hydrogen, C₁-C₁₀ alkyl, C₃-C₈ cycloalkyl, 3- to 10-membered heterocyclyl, C₆-C₁₀ aryl, and 5- to 10-membered heteroaryl; the C₁-C₁₀ alkyl, C₃-C₈ cycloalkyl, 3- to 10-membered heterocycloalkyl, C₆-C₁₀ aryl, and 5- to 10-membered heteroaryl are optionally further substituted by one to two substituents selected from -NR₁₀R₁₁, -OR₁₀, -CN, halogen, C₁-C₃ alkyl, C₃-C₆ cycloalkyl, -C(=O)R₁₀, -C(=O)OR₁₀, -C(=O)NR₁₀R₁₁, -S(=O)₂R₁₀, -S(=O)₂NR₁₀R₁₁, and -NR₁₀C(=O)R₁₁; wherein, R₁₀ and R₁₁ are independently selected from hydrogen and C₁-C₄ alkyl;
Z₁ is N or CR₁₅;
R₁₅ is selected from hydrogen, -CN, C₁-C₄ alkyl, C₃-C₈ cycloalkyl and 3- to 10-membered heterocyclyl;

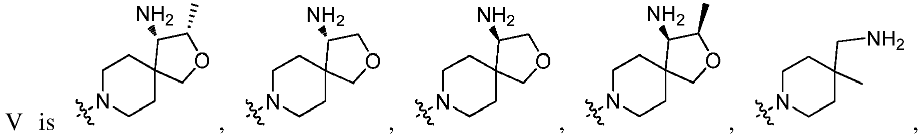

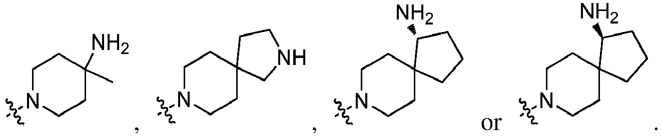

According to an embodiment of the present invention, the compound represented by formula I is selected from the compounds represented by formula Ie: 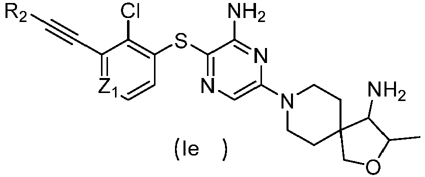 wherein
R₂ is selected from C₁-C₁₀ alkyl, C₃-C₈ cycloalkyl, 3- to 10-membered heterocyclyl, C₆-C₁₀ aryl and 5- to 10-membered heteroaryl; the C₁-C₁₀ alkyl, C₃-C₈ cycloalkyl, 3- to 10-membered heterocyclyl, C₆-C₁₀ aryl, 5- to 10-membered heteroaryl are optionally further substituted by one to two substituents independently selected from halogen, C₁-C₃ alkyl and C₃-C₆ cycloalkyl.
Z₁ is N or CR₁₅;
R₁₅ is selected from hydrogen, C₁-C₄ alkyl and C₃-C₈ cycloalkyl.

According to an embodiment of the present invention, the compound represented by formula I is selected from the compounds below:

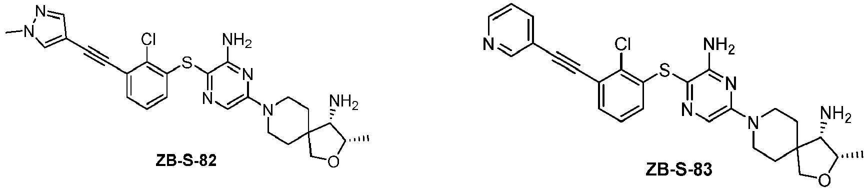

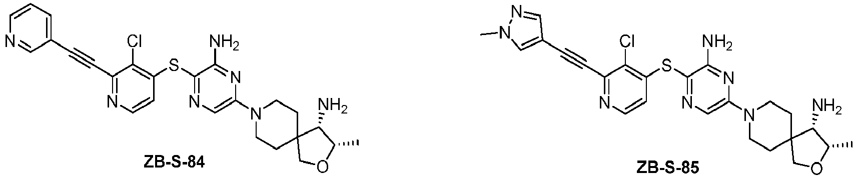

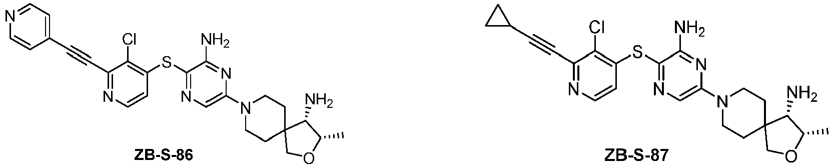

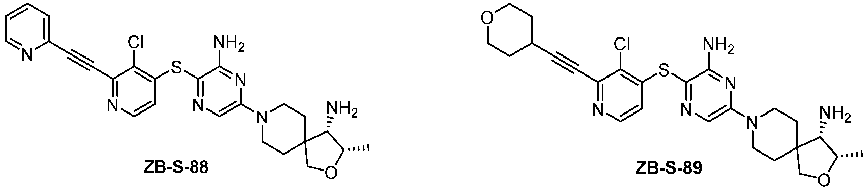

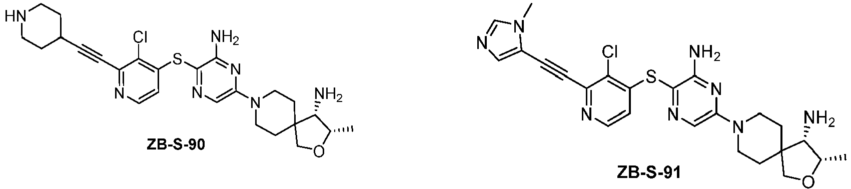

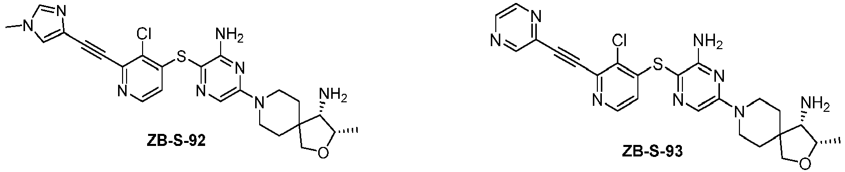

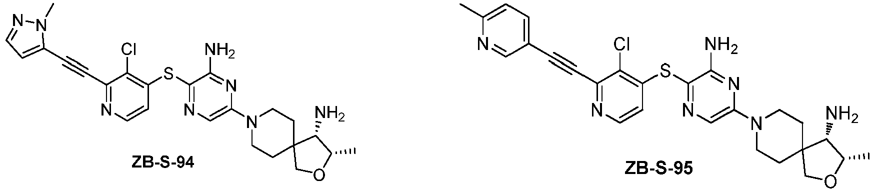

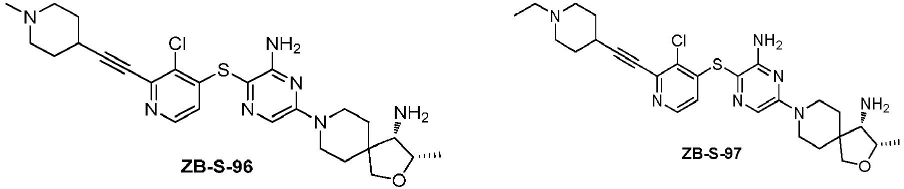

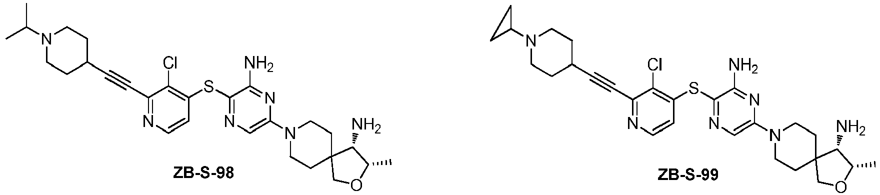

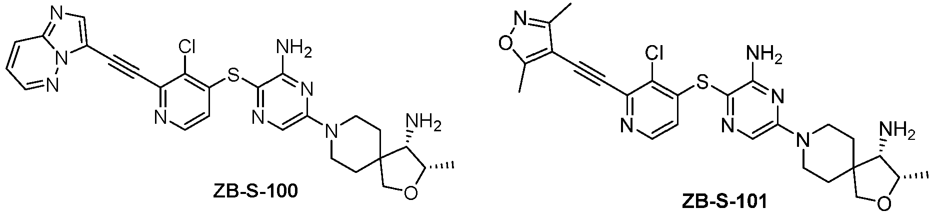

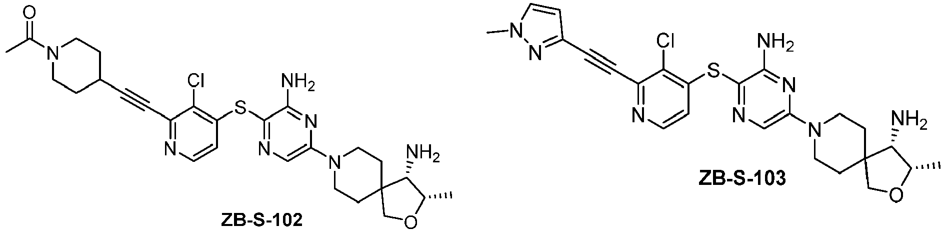

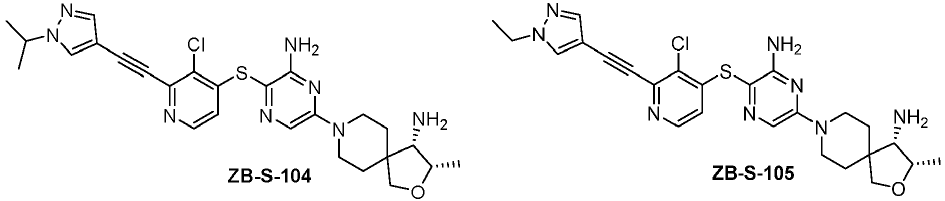

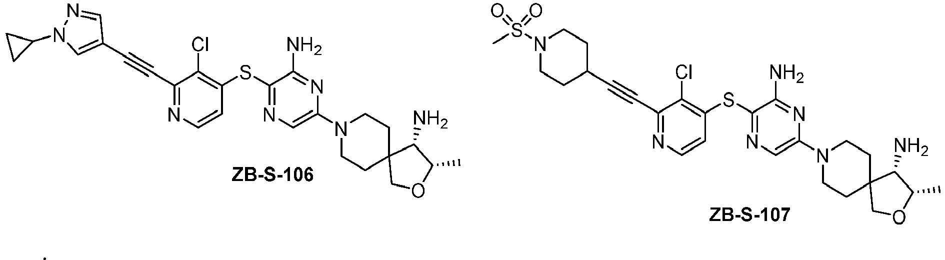

According to an embodiment of the present invention, it provides a pharmaceutical composition comprising a therapeutically effective amount of one or more selected from the above compound, and the pharmaceutically acceptable salt, enantiomer, diastereomer, atropisomer, racemate, polymorph, solvate and isotopically labeled compound thereof, and a pharmaceutically acceptable carrier.

According to an embodiment of the present invention, it provides the use of the compound, or the pharmaceutically acceptable salt, enantiomer, diastereomer, atropisomer, racemate, polymorph, solvate or isotopically labeled compound thereof, or the pharmaceutical composition in preparing a medicament for treating a disease or disorder or condition mediated by SHP2.

According to an embodiment of the present invention, the disease or disorder or condition is selected from Noonan syndrome, leopard skin syndrome, leukemia, neuroblastoma, liver cancer, neuroblastoma, head and neck squamous cell carcinoma, melanoma, breast cancer, esophageal cancer, head and neck tumor, gastric cancer, lung cancer, colon cancer, anaplastic large cell lymphoma, and glioblastoma.

According to an embodiment of the present invention, the disease or disorder or condition is selected from: non-small cell lung cancer, esophageal cancer and head and neck tumor.

Furthermore, the leukemia is acute myeloid leukemia (AML), and the lung cancer is non-small cell lung cancer (NSCLC).

According to an embodiment of the present invention, it provides the use of the compound, or the pharmaceutically acceptable salt, enantiomer, diastereomer, atropisomer, racemate, polymorph, solvate or isotopically labeled compound thereof, or the pharmaceutical composition in preparing a medicament as a SHP2 inhibitor.

According to an embodiment of the present invention, it provides a method for preventing and/or treating a disease or disorder or condition mediated by SHP2, which includes administering to a patient a therapeutically effective dose of one or more selected from the above compound, and the pharmaceutically acceptable salt, enantiomer, diastereomer, atropisomer, racemate, polymorph, solvate and isotopically labeled compound thereof, or the above pharmaceutical composition.

### Examples

The following examples are only used to illustrate the technical solution of the present invention without limiting the scope of the present invention. The compounds of the present invention can be synthesized in a similar manner with reference to the preparation methods in the following examples.

### Synthesis of intermediate M-1-4

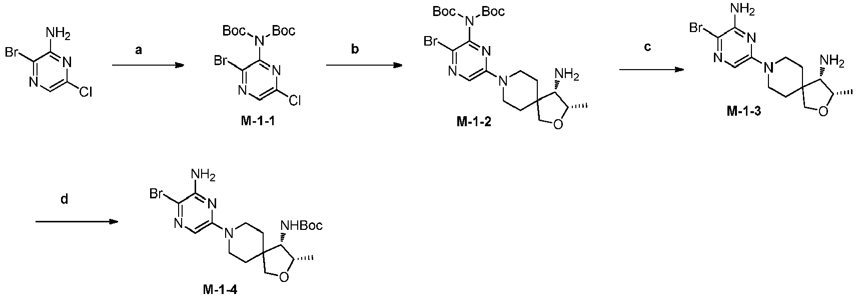

(a) 2-Amino-3-bromo-6-chloropyrazine (3 g, 14.39 mmol) and di-tert-butyl dicarbonate (15.71 g, 71.96 mmol) were dissolved in 50 mL of dichloromethane. 4-Dimethylaminopyridine (879 mg, 7.20 mmol) was added to the above reaction system at room temperature and refluxed for 2h. The solvent was rotary evaporated off, and the residue was separated by column chromatography to obtain an intermediate M-1-1. ¹H NMR (400 MHz, chloroform-d) δ 8.34 (s, 1H), 1.42 (s, 18H).
(b) The intermediate **M-1-1** (1g, 2.45 mmol), (3S,4S)-3-methyl-2-oxa-8-azaspiro[4,5]decan-4-amine dihydrochloride (714 mg, 2.94 mmol) and potassium phosphate (2.59 g, 12.23 mmol) were dispersed in 20 mL of N-methylpyrrolidone, and reacted at 130 °C for 6 hours. The reaction system was cooled to room temperature. Saturated brine was added, and the mixture was extracted with ethyl acetate. The organic phase was washed with saturated brine, dried over anhydrous sodium sulfate and concentrated. The residue was separated by column chromatography to obtain a crude intermediate **M-1-2**. HPLC-MS: [M+H]⁺=542.36/544.02.
(c) The crude intermediate **M-1-2** (300 mg, 0.55 mmol) was dissolved in 10 mL of dichloromethane. 2 mL of trifluoroacetic acid was added to the above system at room temperature, and reacted at room temperature for 2h, and the solvent was rotary evaporated off. The residue was separated by column chromatography to obtain an intermediate **M-1-3**. HPLC-MS: [M+H]⁺=342.10/344.08.
(d) The intermediate **M-1-3** (80 mg, 0.23 mmol) was dissolved in 10 mL of methylene chloride. Di-tert-butyl dicarbonate (102 mg, 0.47 mmol) was added to the above system, and stirred at room temperature for 48 h. The solvent was rotary evaporated off, and the residue was separated by column chromatography to obtain an intermediate **M-1-4**. HPLC-MS: [M+H]⁺ =442.23/444.16. ¹H NMR (400 MHz, methanol-*d*₄) δ 7.17 (s, 1H), 4.28 - 4.19 (m, 1H), 3.98 - 3.91 (m, 1H), 3.71 (d, *J* = 8.7 Hz, 1H), 3.68 - 3.34 (m, 5H), 1.83 - 1.49 (m, 4H), 1.45 (s, 9H), 1.14 (d, *J* = 6.3 Hz, 3H).

### Synthesis of intermediate M-2-2

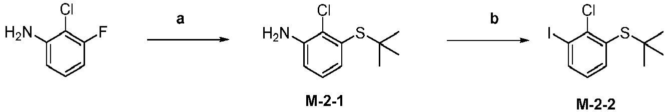

(a) 2-Chloro-3-fluoroaniline (3 g, 20.61 mmol) was dissolved in 50mL of DMF, and cesium carbonate (10.07 g, 30.92 mmol) and tert-butylmercaptan (6.97 mL, 61.83 mmol) were added to the above system, and reacted at 120 °C for 36 h. The reaction system was cooled to room temperature. Saturated brine was added, and the mixture was extracted with ethyl acetate (100 mL*3), and the ethyl acetate layers were combined. The organic phase was washed with saturated brine, dried over anhydrous sodium sulfate and concentrated. The residue was separated by column chromatography to obtain an intermediate **M-2-1**. ¹H NMR (400 MHz, chloroform-d) δ 7.04 (dd, *J* = 7.6, 2.0 Hz, 1H), 7.01 (t, *J* = 7.6 Hz, 1H), 6.77 (dd, *J* = 7.5, 2.0 Hz, 1H), 4.16 (s, 2H), 1.33 (s, 9H).
(b) The intermediate M-2-1 (3 g, 13.91 mmol) was dissolved in 30 mL of concentrated hydrochloric acid, and an aqueous solution of sodium nitrite (1.15 g, 16.69 mmol) was added to the above system at 0 °C. After 30 minutes, an aqueous solution of potassium iodide (4.62 g, 27.81 mmol) was added to the above system, and reacted for 30 minutes. The reaction system was restored to room temperature. Saturated brine was added. The mixture was extracted with ethyl acetate, and the ethyl acetate layers were combined. The organic phase was washed with saturated brine, dried over anhydrous sodium sulfate and concentrated. The residue was separated by column chromatography to obtain an intermediate **M-2-2**. ¹H NMR (400 MHz, chloroform-d) δ 7.86 (dd, *J* = 7.8, 1.5 Hz, 1H), 7.62 (dd, *J* = 7.8, 1.5 Hz, 1H), 6.91 (t, *J* = 7.8 Hz, 1H), 1.34 (s, 9H).

### Synthesis of intermediate M-3-1

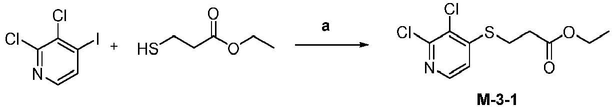

2,3-Dichloro-4-iodopyridine (5 g, 18.25 mmol) and diisopropylethylamine (9.07 mL, 54.74 mmol) were dissolved in 50 mL of 1,4-dioxane, and Pd₂(dba)₃ (826 mg, 0.91 mmol) and Xantphos (1.06 g, 1.82 mmol) were added to the above system. The atmosphere was replaced with argon, and a solution of ethyl 3-mercaptopropionate (2.08 mL, 16.42 mmol) in dioxane was slowly and dropwise added at 100 °C. After the dropwise addition was completed, the resultant was reacted for 2 h. The solvent was rotary evaporated off, and the residue was separated by column chromatography to obtain an intermediate **M-3-1**. HPLC-MS: [M+H]⁺ =279.79/281.68/283.62. ¹H NMR (400 MHz, chloroform-d) δ 8.15 (d, *J* = 5.3 Hz, 1H), 7.03 (d, *J* = 5.3 Hz, 1H), 4.19 (q, *J* = 7.2 Hz, 2H), 3.25 (t, *J* = 7.4 Hz, 2H), 2.74 (t, *J* = 7.4 Hz, 2H), 1.28 (t, *J* = 7.2 Hz, 3H).

### Example 1

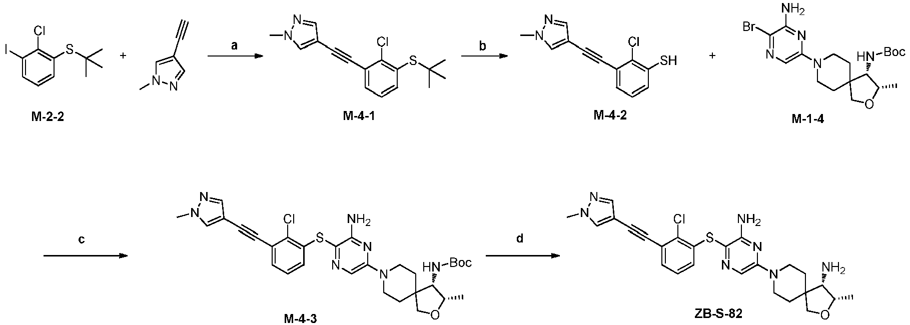

(a) The intermediate **M-2-**2 (300 mg, 0.92 mmol) and N-methyl-4-ynylpyrazole (117 mg, 1.10 mmol) were dissolved in a mixed solvent of Et₃N/DMF (5 mL/10 mL), and Pd(PPh₃)₂Cl₂ (64 mg, 0.092 mmol) and CuI (35 mg, 0.18 mmol) were added to the above system. The atmosphere was replaced with argon, and the reaction was carried out at 80 °C for 2 h. The reaction solution was cooled to room temperature. 50 mL of saturated brine was added, and the mixture was extracted three times with ethyl acetate (30 mL*3), and the ethyl acetate layers were combined. The organic phase was washed with saturated brine, dried over anhydrous sodium sulfate and concentrated. The residue was separated by column chromatography to obtain an intermediate **M-4-1**. HPLC-MS: [M+H]⁺ =304.90/306.74. ¹H NMR (400 MHz, chloroform-d) δ 7.67 (s, 1H), 7.61 (s, 1H), 7.58 (dd, *J* = 7.7, 1.7 Hz, 1H), 7.51 (dd, *J* = 7.7, 1.7 Hz, 1H), 7.18 (t, *J* = 7.7 Hz, 1H), 3.92 (s, 3H), 1.34 (s, 9H).
(b) The intermediate **M-4-1** (200 mg, 0.66 mmol) was dissolved in 15 mL of a ultra-dry toluene, and AlCl₃ (351 mg, 2.62 mmol) was added to the above system at room temperature. After 30 min, silica gel was added to mix the sample, and the product was separated by column chromatography to obtain a crude intermediate **M-4-2**. HPLC-MS: [M+H]⁺=248.84/250.67.
(c) The intermediate **M-4-2** (34 mg, 0.14 mmol), **M-1-4** (50 mg, 0.11 mmol), and diisopropylethylamine (56 µL, 0.34 mmol) were dissolved in 2 mL of 1,4-dioxane, and Pd₂(dba)₃ (10 mg, 0.011 mmol) and Xantphos (13 mg, 0.023 mmol) were added to the above system. The atmosphere was replaced with argon, and the reaction was carried out under microwave for 2 h at 130 °C. The solvent was rotary evaporated off, and the residue was separated by column chromatography to obtain an intermediate **M-4-3**. HPLC-MS: [M+H]⁺ =610.32/612.11.
(d) The intermediate **M-4-3** was dissolved in 5 mL of methylene chloride, and 1 mL of trifluoroacetic acid was added to the above system at room temperature. The resultant was reacted at room temperature for 2 h, and the solvent was rotary evaporated off. The residue was separated by column chromatography to obtain **ZB-S-82.** HPLC-MS: [M+H]⁺=510.05/511.89. ¹H NMR (400 MHz, methanol-*d*₄) δ 7.89 (s, 1H), 7.66 (s, 1H), 7.56 (s, 1H), 7.29 (dd, *J* = 7.9, 1.5 Hz, 1H), 7.10 (t, *J* = 7.9 Hz, 1H), 6.64 (dd, *J* = 7.9, 1.5 Hz, 1H), 4.28 - 4.19 (m, 1H), 4.11 - 3.97 (m, 2H), 3.91 (s, 3H), 3.87 (d, *J* = 8.7 Hz, 1H), 3.72 (d, *J* = 8.7 Hz, 1H), 3.39 - 3.20 (m, 2H), 3.03 (d, *J* = 4.9 Hz, 1H), 1.86 - 1.59 (m, 4H), 1.22 (d, *J* = 6.5 Hz, 3H).

### Example 2

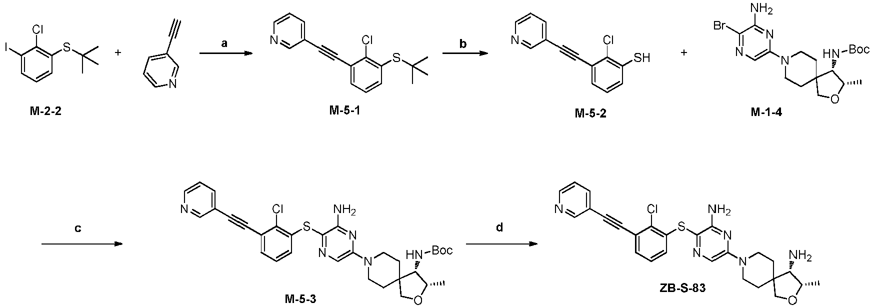

(a) The intermediate **M-2-2** (300 mg, 0.92 mmol) and 3-ethynylpyridine (114 mg, 1.10 mmol) were dissolved in a mixed solvent of Et₃N/DMF (5 mL/10 mL), and Pd(PPh₃)₂Cl₂ (64 mg, 0.092 mmol) and CuI (35 mg, 0.18 mmol) were added to the above system. The atmosphere was replaced with argon, and the reaction was carried out at 80 °C for 2 h. The reaction solution was cooled to room temperature. 50 mL of saturated brine was added, and the mixture was extracted three times with ethyl acetate (30 mL*3), and the ethyl acetate layers were combined. The organic phase was washed with saturated brine, dried over anhydrous sodium sulfate and concentrated. The residue was separated by column chromatography to obtain an intermediate **M-5-1**. HPLC-MS: [M+H]⁺ =301.90/303.74. ¹H NMR (400 MHz, chloroform-d) δ 8.82 - 8.78 (m, 1H), 8.57 (dd, *J* = 4.9, 1.7 Hz, 1H), 7.85 (dt, *J =* 7.9, 1.9 Hz, 1H), 7.65 (dd, *J* = 7.7, 1.7 Hz, 1H), 7.58 (dd, *J* = 7.7, 1.7 Hz, 1H), 7.30 (ddd, *J* = 7.9, 4.9, 0.9 Hz, 1H), 7.23 (t, *J* = 7.7 Hz, 1H), 1.36 (s, 9H).
(b) The intermediate **M-5-1** (200 mg, 0.66 mmol) was dissolved in 15 mL of ultra-dry toluene, and AlCl₃ (351 mg, 2.62 mmol) was added to the above system at room temperature. After 30 min, silica gel was added to mix the sample, and the product was separated by column chromatography to obtain a crude intermediate **M-5-2**. HPLC-MS: [M+H]⁺=245.79/247.63.
(c) The intermediate **M-5-2** (33 mg, 0.14 mmol), **M-1-4** (50 mg, 0.11 mmol), and diisopropylethylamine (56 µL, 0.34 mmol) were dissolved in 2 mL of 1,4-dioxane, and Pd₂(dba)₃ (10 mg, 0.011 mmol) and Xantphos (13 mg, 0.023 mmol) were added to the above system. The atmosphere was replaced with argon, and the reaction was carried out under microwave for 2 h at 130 °C. The solvent was rotary evaporated off, and the residue was separated by column chromatography to obtain an intermediate **M-5-3**. HPLC-MS: [M+H]⁺ =607.27/609.06.
(d) The intermediate **M-5-3** was dissolved in 5 mL of methylene chloride, and 1 mL of trifluoroacetic acid was added to the above system at room temperature. The resultant was reacted at room temperature for 2 h, and the solvent was rotary evaporated off. The residue was separated by column chromatography to obtain **ZB-S-83**. HPLC-MS: [M+H]⁺=507.10/508.89. ¹H NMR (400 MHz, methanol-*d*₄) δ 8.73 (dd, *J* = 2.1, 0.9 Hz, 1H), 8.56 (dd, *J* = 5.0, 1.7 Hz, 1H), 8.01 (dt, *J* = 7.9, 1.9 Hz, 1H), 7.59 (s, 1H), 7.50 (ddd, *J* = 8.0, 5.0, 1.0 Hz, 1H), 7.42 (dd, *J* = 7.9, 1.5 Hz, 1H), 7.17 (t, *J* = 7.9 Hz, 1H), 6.73 (dd, *J* = 7.9, 1.5 Hz, 1H), 4.33 - 4.08 (m, 3H), 3.94 (d, *J* = 8.9 Hz, 1H), 3.81 (d, *J* = 8.9 Hz, 1H), 3.38 - 3.10 (m, 3H), 1.88 - 1.62 (m, 4H), 1.27 (d, *J* = 6.5 Hz, 3H).

### Example 3

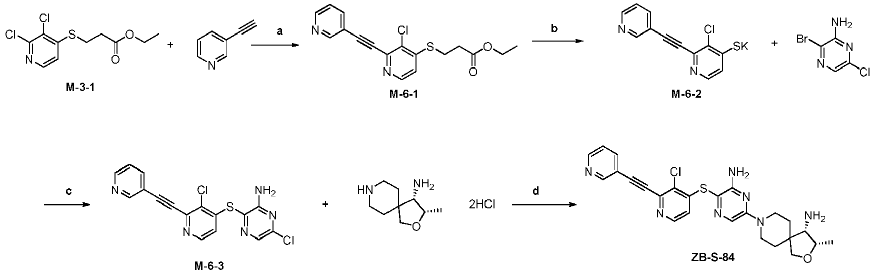

(a) The intermediate **M-3-1** (400 mg, 1.43 mmol), 3-ethynylpyridine (162 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-6-1**. HPLC-MS: [M+H]⁺ =346.94/348.77. ¹H NMR (400 MHz, chloroform-*d*) δ 9.12 - 8.78 (m, 1H), 8.76 - 8.50 (m, 1H), 8.38 (d, *J* = 5.1 Hz, 1H), 7.92 (d, *J* = 7.9 Hz, 1H), 7.42 - 7.28 (m, 1H), 7.08 (d, *J* = 5.1 Hz, 1H), 4.20 (q, *J* = 7.2 Hz, 1H), 3.27 (t, *J* = 7.4 Hz, 2H), 2.76 (t, *J* = 7.4 Hz, 2H), 1.29 (t, *J* = 7.2 Hz, 2H).
(b) The intermediate **M-6-1** (200 mg, 0.58 mmol) was dissolved in 15 mL of anhydrous tetrahydrofuran, and the atmosphere was replaced with argon. Potassium tert-butoxide (78 mg, 0.69 mmol) was added to the above system at room temperature, and the reaction was carried out for 1 h. The solvent was rotary evaporated off to obtain an intermediate **M-6-2**, which was directly used in the next step without purification. HPLC-MS: [M+H]⁺=246.71/266.98.
(c) The intermediate **M-6-2** (165 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-6-3**. HPLC-MS: [M+H]⁺ =373.88/375.86/377.99.
(d) The intermediate **M-6-3** (60 mg, 0.16 mmol), (3S,4S)-3-methyl-2-oxa-8-azaspiro[4,5]decan-4-amine dihydrochloride (CAS: 2055761-19-6, 47 mg, 0.19 mmol), and diisopropylethylamine (264 µL, 1.60 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-84**. HPLC-MS: [M+H]⁺=508.11/509.90. ¹H NMR (400 MHz, methanol-*d*₄) δ 8.83 - 8.76 (m, 1H), 8.61 (dd, *J* = 4.9, 1.6 Hz, 1H), 8.19 (d, *J* = 5.4 Hz, 1H), 8.13 - 8.04 (m, 1H), 7.64 (s, 1H), 7.53 (dd, *J* = 8.0, 4.9 Hz, 1H), 6.68 (d, *J* = 5.4 Hz, 1H), 4.34 - 4.13 (m, 3H), 3.94 (d, *J* = 8.9 Hz, 1H), 3.82 (d, *J* = 8.9 Hz, 1H), 3.34 - 3.11 (m, 3H), 1.87 - 1.62 (m, 4H), 1.28 (d, *J* = 6.5 Hz, 3H).

### Example 4

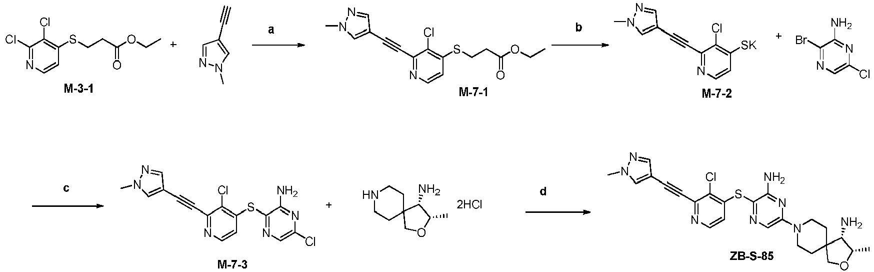

(a) The intermediate **M-3-1** (400 mg, 1.43 mmol), and N-methyl-4-ynylpyrazole (167 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-7-1**. HPLC-MS: [M+H]⁺=349.98/351.82. ¹H NMR (400 MHz, chloroform-d) δ 8.32 (d, *J* = 5.3 Hz, 1H), 7.72 (s, 1H), 7.66 (s, 1H), 7.01 (d, *J* = 5.3 Hz, 1H), 4.19 (q, *J* = 7.1 Hz, 2H), 3.92 (s, 3H), 3.25 (t, *J* = 7.4 Hz, 2H), 2.74 (t, *J* = 7.4 Hz, 2H), 1.28 (t, *J* = 7.1 Hz, 3H).
(b) The intermediate **M-7-1** (200 mg, 0.57 mmol) was dissolved in 15 mL of anhydrous tetrahydrofuran, and the atmosphere was replaced with argon. Potassium tert-butoxide (77 mg, 0.69 mmol) was added to the above system at room temperature, and the reaction was carried out for 1 h. The solvent was rotary evaporated off to obtain an intermediate **M-7-2**, which was directly used in the next step without purification. HPLC-MS: [M+H]⁺=249.85/251.69.
(c) The intermediate **M-7-2** (165 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-7-3**. HPLC-MS: [M+H]⁺ =376.93/378.76/380.70.
(d) The intermediate **M-7-3** (65 mg, 0.17 mmol), (3S,4S)-3-methyl-2-oxa-8-azaspiro[4,5]decan-4-amine dihydrochloride (CAS: 2055761-19-6, 50 mg, 0.21 mmol), and diisopropylethylamine (285 µL, 1.72 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-85**. HPLC-MS: [M+H]⁺=511.16/513.00. ¹H NMR (400 MHz, methanol-*d*₄) δ 8.12 (d, *J* = 5.4 Hz, 1H), 7.99 (s, 1H), 7.74 (s, 1H), 7.62 (s, 1H), 6.60 (d, *J* = 5.4 Hz, 1H), 4.31 - 4.19 (m, 1H), 4.16 - 4.02 (m, 2H), 3.93 (s, 3H), 3.89 (d, *J* = 8.7 Hz, 1H), 3.74 (d, *J* = 8.7 Hz, 1H), 3.42 - 3.21 (m, 2H), 3.06 (d, *J* = 4.9 Hz, 1H), 1.91 - 1.52 (m, 4H), 1.23 (d, *J* = 6.4 Hz, 3H).

### Example 5

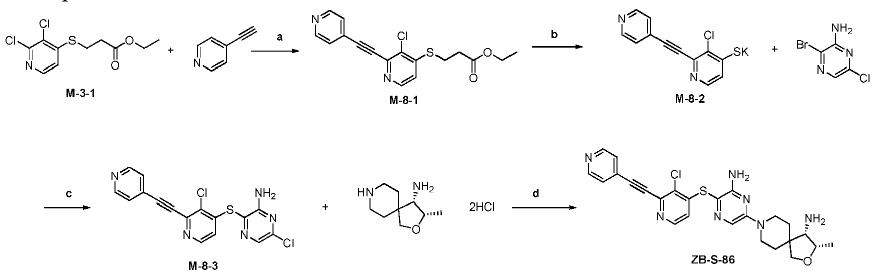

(a) The intermediate **M-3-1** (400 mg, 1.43 mmol), and 4-ethynylpyridine (162 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-8-1**. HPLC-MS: [M+H]⁺ =346.94/349.11. ¹H NMR (400 MHz, chloroform-d) δ 8.72 - 8.55 (m, 2H), 8.37 (d, *J* = 5.3 Hz, 1H), 7.51 - 7.43 (m, 2H), 7.09 (d, *J* = 5.3 Hz, 1H), 4.19 (q, *J* = 7.1 Hz, 2H), 3.26 (t, *J* = 7.4 Hz, 2H), 2.75 (t, *J* = 7.4 Hz, 2H), 1.28 (t, *J* = 7.1 Hz, 3H).
(b) The intermediate **M-8-1** (200 mg, 0.58 mmol) was dissolved in 15 mL of anhydrous tetrahydrofuran, and the atmosphere was replaced with argon. Potassium tert-butoxide (78 mg, 0.69 mmol) was added to the above system at room temperature, and the reaction was carried out for 1 h. The solvent was rotary evaporated off to obtain an intermediate **M-8-2**, which was directly used in the next step without purification. HPLC-MS: [M+H]⁺=246.85/249.03.
(c) The intermediate **M-8-2** (165 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-8-3**. HPLC-MS: [M+H]⁺ =373.88/375.77/377.65.
(d) The intermediate **M-8-3** (59 mg, 0.16 mmol), (3S,4S)-3-methyl-2-oxa-8-azaspiro[4,5]decan-4-amine dihydrochloride (CAS: 2055761-19-6, 46 mg, 0.19 mmol), and diisopropylethylamine (261 µL, 1.57 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-86.** HPLC-MS: [M+H]⁺=508.16/510.39. ¹H NMR (400 MHz, DMSO-*d*₆) δ 8.74 - 8.66 (m, 2H), 8.30 (d, *J* = 5.3 Hz, 1H), 7.68 (s, 1H), 7.64 - 7.58 (m, 2H), 6.62 (d, *J* = 5.3 Hz, 1H), 6.31 (s, 2H), 4.12 - 4.02 (m, 1H), 3.94 - 3.79 (m, 2H), 3.68 (d, *J* = 8.4 Hz, 1H), 3.50 (d, *J* = 8.4 Hz, 1H), 3.46 - 3.26 (m, 2H), 2.93 (d, *J* = 5.0 Hz, 1H), 1.80 - 1.41 (m, 4H), 1.09 (d, *J* = 6.4 Hz, 3H).

### Example 6

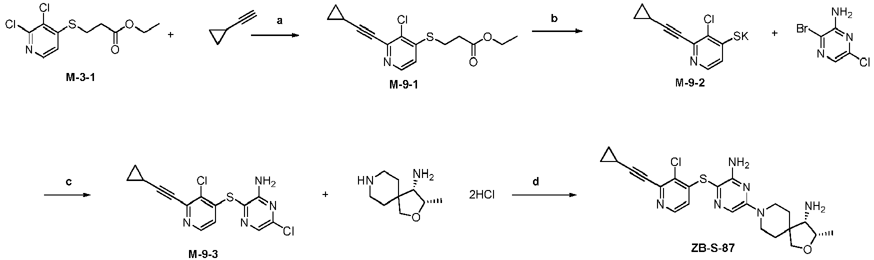

(a) The intermediate **M-3-1** (400 mg, 1.43 mmol), and cyclopropylacetylene (167 mg, 7.14 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-9-1**. HPLC-MS: [M+H]⁺ =309.88/311.67. ¹H NMR (400 MHz, chloroform-*d*) δ 8.25 (d, *J* = 5.3 Hz, 1H), 6.96 (d, *J* = 5.3 Hz, 1H), 4.18 (q, *J* = 7.1 Hz, 2H), 3.22 (t, *J* = 7.4 Hz, 2H), 2.72 (t, *J* = 7.4 Hz, 2H), 1.57 - 1.49 (m, 1H), 1.27 (t, *J* = 7.1 Hz, 3H), 0.96 - 0.91 (m, 4H).
(b) The intermediate **M-9-1** (200 mg, 0.65 mmol) was dissolved in 15 mL of anhydrous tetrahydrofuran, and the atmosphere was replaced with argon. Potassium tert-butoxide (87 mg, 0.77 mmol) was added to the above system at room temperature, and the reaction was carried out for 1 h. The solvent was rotary evaporated off to obtain an intermediate **M-9-2**, which was directly used in the next step without purification.
(c) The intermediate **M-9-2** (142 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-9-3**. HPLC-MS: [M+H]⁺ =336.92/338.76/340.79.
(d) The intermediate **M-9-3** (60 mg, 0.18 mmol), (3S,4S)-3-methyl-2-oxa-8-azaspiro[4,5]decan-4-amine dihydrochloride (52 mg, 0.21 mmol), and diisopropylethylamine (294 µL, 1.78 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-87.** HPLC-MS: [M+H]⁺=471.18/473.29. ¹H NMR (400 MHz, methanol-*d*₄) δ 8.04 (d, *J* = 5.4 Hz, 1H), 7.61 (s, 1H), 6.55 (d, *J* = 5.4 Hz, 1H), 4.30 - 4.19 (m, 1H), 4.19 - 4.03 (m, 2H), 3.89 (d, *J* = 8.8 Hz, 1H), 3.75 (d, *J* = 8.8 Hz, 1H), 3.39 - 3.19 (m, 2H), 3.10 (d, *J* = 4.8 Hz, 1H), 1.88 - 1.53 (m, 4H), 1.24 (d, *J* = 6.5 Hz, 3H), 1.06 - 0.97 (m, 2H), 0.93 - 0.84 (m, 2H).

### Example 7

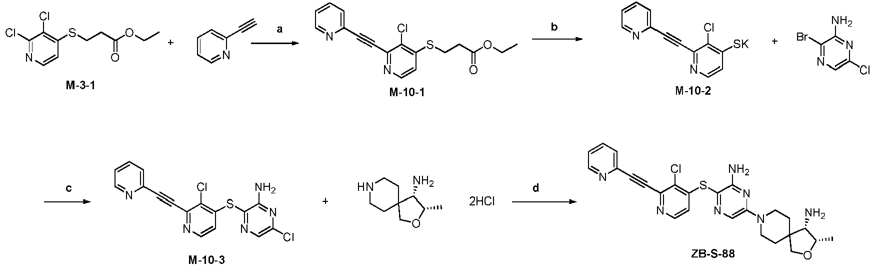

(a) The intermediate **M-3-1** (400 mg, 1.43 mmol), and 2-ethynylpyridine (162 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-10-1.** HPLC-MS: [M+H]⁺ =346.94/349.11.
(b) The intermediate **M-10-1** (200 mg, 0.58 mmol) was dissolved in 15 mL of anhydrous tetrahydrofuran, and the atmosphere was replaced with argon. Potassium tert-butoxide (78 mg, 0.69 mmol) was added to the above system at room temperature, and the reaction was carried out for 1 h. The solvent was rotary evaporated off to obtain an intermediate **M-10-2,** which was directly used in the next step without purification. HPLC-MS: [M+H]⁺=246.85/249.03.
(c) The intermediate **M-10-2** (164 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-10-3.** HPLC-MS: [M+H]⁺ =373.88/375.77/377.80.
(d) The intermediate **M-10-3** (40 mg, 0.11 mmol), (3S,4S)-3-methyl-2-oxa-8-azaspiro[4,5]decan-4-amine dihydrochloride (31 mg, 0.13 mmol), and diisopropylethylamine (177 µL, 1.07 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-88.** HPLC-MS: [M+H]⁺=508.11/510.14. ¹H NMR (400 MHz, methanol-*d*₄) δ 8.65 - 8.59 (m, 1H), 8.20 (d, *J* = 5.4 Hz, 1H), 7.93 (td, *J* = 7.8, 1.8 Hz, 1H), 7.81 - 7.74 (m, 1H), 7.64 (s, 1H), 7.51 (ddd, *J* = 7.7, 4.9, 1.2 Hz, 1H), 6.68 (d, *J* = 5.4 Hz, 1H), 4.34 - 4.19 (m, 3H), 3.97 (d, *J* = 9.1 Hz, 1H), 3.85 (d, *J* = 9.1 Hz, 1H), 3.35 (d, *J* = 4.3 Hz, 1H), 3.29 - 3.12 (m, 2H), 1.89 - 1.63 (m, 4H), 1.30 (d, *J* = 6.5 Hz, 3H).

### Example 8

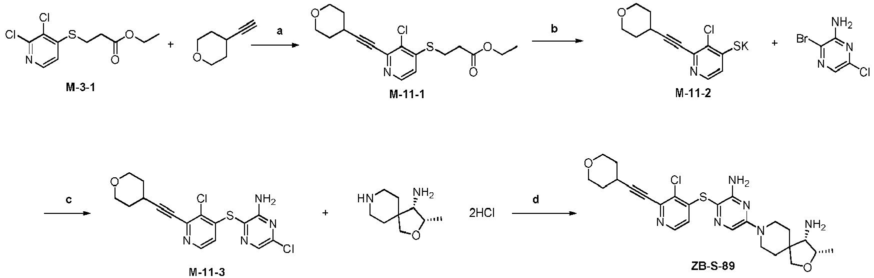

(a) The intermediate **M-3-1** (400 mg, 1.43 mmol), and 2-ethynylpyran (173 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-11-1.** HPLC-MS: [M+H]⁺ =354.05/355.84. ¹H NMR (400 MHz, chloroform-*d*) δ 8.29 (d, *J* = 5.3 Hz, 1H), 7.01 (d, *J* = 5.3 Hz, 1H), 4.19 (q, *J* = 7.2 Hz, 2H), 3.97 (ddd, *J* = 11.7, 6.3, 3.6 Hz, 2H), 3.59 (ddd, *J* = 11.4, 7.9, 3.1 Hz, 2H), 3.24 (t, *J* = 7.5 Hz, 2H), 2.98 (tt, *J* = 8.2, 4.2 Hz, 1H), 2.73 (t, *J* = 7.5 Hz, 2H), 2.01 - 1.89 (m, 2H), 1.89 - 1.74 (m, 2H), 1.28 (t, *J* = 7.2 Hz, 3H).
(b) The intermediate **M-11-1** (250 mg, 0.71 mmol) was dissolved in 15 mL of anhydrous tetrahydrofuran, and the atmosphere was replaced with argon. Potassium tert-butoxide (95 mg, 0.85 mmol) was added to the above system at room temperature, and the reaction was carried out for 1 h. The solvent was rotary evaporated off to obtain an intermediate **M-11-2,** which was directly used in the next step without purification. HPLC-MS: [M+H]⁺=253.91/255.75.
(c) The intermediate **M-11-2** (168 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-11-3.** HPLC-MS: [M+H]⁺ =380.99/382.98/384.86.
(d) The intermediate **M-11-3** (70 mg, 0.18 mmol), (3S,4S)-3-methyl-2-oxa-8-azaspiro[4,5]decan-4-amine dihydrochloride (53 mg, 0.22 mmol), and diisopropylethylamine (303 µL, 1.83 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-89.** HPLC-MS: [M+H]⁺=514.25/516.37. ¹H NMR (400 MHz, DMSO-*d*₆) δ 8.18 (d, *J* = 5.2 Hz, 1H), 7.67 (s, 1H), 6.49 (d, *J* = 5.2 Hz, 1H), 6.27 (s, 2H), 4.15 - 4.07 (m, 1H), 4.05 - 3.90 (m, 2H), 3.86 - 3.78 (m, 2H), 3.76 (d, *J* = 8.6 Hz, 1H), 3.56 (d, *J* = 8.6 Hz, 1H), 3.54 - 3.45 (m, 2H), 3.33 - 3.15 (m, 2H), 3.10 (d, *J* = 5.0 Hz, 1H), 3.03 (tt, *J* = 8.5, 4.2 Hz, 1H), 1.93 - 1.83 (m, 2H), 1.76 - 1.40 (m, 6H), 1.13 (d, *J* = 6.4 Hz, 3H).

### Example 9

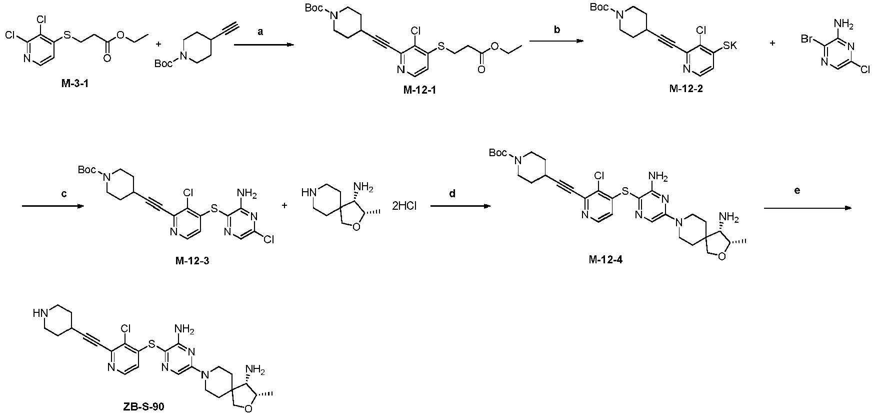

(a) The intermediate **M-3-1** (400 mg, 1.43 mmol), and 1-Boc-4-ethynylpiperidine (330 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-12-1.** HPLC-MS: [M+H]⁺=453.02/454.81. ¹H NMR (400 MHz, chloroform-d) δ 8.29 (d, *J* = 5.4 Hz, 1H), 7.01 (d, *J* = 5.4 Hz, 1H), 4.19 (q, *J* = 7.1 Hz, 2H), 3.71 (ddd, *J* = 12.9, 7.2, 3.5 Hz, 3H), 3.33 (ddd, *J* = 13.5, 7.7, 3.6 Hz, 2H), 3.24 (t, *J* = 7.4 Hz, 2H), 2.93 (tt, *J* = 7.6, 4.0 Hz, 1H), 2.73 (t, *J* = 7.4 Hz, 2H), 1.96 - 1.82 (m, 2H), 1.81 - 1.68 (m, 2H), 1.46 (s, 9H), 1.28 (t, *J* = 7.1 Hz, 3H).
(b) The intermediate **M-12-1** (300 mg, 0.66 mmol) was dissolved in 15 mL of anhydrous tetrahydrofuran, and the atmosphere was replaced with argon. Potassium tert-butoxide (89 mg, 0.79 mmol) was added to the above system at room temperature, and the reaction was carried out for 1 h. The solvent was rotary evaporated off to obtain an intermediate **M-12-2,** which was directly used in the next step without purification.
(c) The intermediate **M-12-2** (224 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-12-3.** HPLC-MS: [M+H]⁺ =480.01/481.89/483.54.
(d) The intermediate **M-12-3** (80 mg, 0.17 mmol), (3S,4S)-3-methyl-2-oxa-8-azaspiro[4,5]decan-4-amine dihydrochloride (53 mg, 0.20 mmol), and diisopropylethylamine (276 µL, 1.67 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **M-12-4.** HPLC-MS: [M+H]⁺=614.39/616.17.
(e) The intermediate **M-12-4** was dissolved in 5 mL of methylene chloride, and 1 mL of trifluoroacetic acid was added to the above system at room temperature. The resultant was reacted at room temperature for 2 h, and the solvent was rotary evaporated off. The residue was separated by column chromatography to obtain **ZB-S-90.** HPLC-MS: [M+H]⁺=514.21/515.95. ¹H NMR (400 MHz, methanol-*d*₄) δ 8.11 (d, *J* = 5.4 Hz, 1H), 7.62 (s, 1H), 6.63 (d, *J* = 5.4 Hz, 1H), 4.29 - 4.20 (m, 1H), 4.16 - 4.00 (m, 2H), 3.88 (d, *J* = 8.7 Hz, 1H), 3.73 (d, *J* = 8.7 Hz, 1H), 3.43 - 3.22 (m, 4H), 3.19 - 3.01 (m, 4H), 2.22 - 2.09 (m, 2H), 2.00 - 1.88 (m, 2H), 1.87 - 1.56 (m, 4H), 1.23 (d, *J* = 6.5 Hz, 3H).

### Example 10

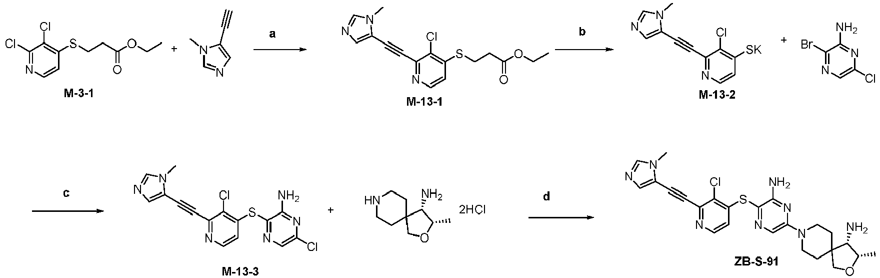

(a) The intermediate **M-3-1** (400 mg, 1.43 mmol), and 5-ethynyl-1-methyl-1H-imidazole (167 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-13-1.** HPLC-MS: [M+H]⁺=349.38/351.77. ¹H NMR (400 MHz, chloroform-*d*) δ 8.35 (d, *J* = 5.3 Hz, 1H), 7.55 (br, 2H), 7.05 (d, *J* = 5.3 Hz, 1H), 4.20 (q, *J* = 7.2 Hz, 2H), 3.80 (s, 3H), 3.27 (t, *J* = 7.4 Hz, 2H), 2.75 (t, *J* = 7.4 Hz, 2H), 1.29 (t, *J* = 7.2 Hz, 3H).
(b) The intermediate **M-13-1** (200 mg, 0.57 mmol) was dissolved in 15 mL of anhydrous tetrahydrofuran, and the atmosphere was replaced with argon. Potassium tert-butoxide (77 mg, 0.69 mmol) was added to the above system at room temperature, and the reaction was carried out for 1 h. The solvent was rotary evaporated off to obtain an intermediate **M-13-2,** which was directly used in the next step without purification.
(c) The intermediate **M-13-2** (165 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-13-3.** HPLC-MS: [M+H]⁺ =376.95/378.66/380.73.
(d) The intermediate **M-13-3** (70 mg, 0.19 mmol), (3S,4S)-3-methyl-2-oxa-8-azaspiro[4,5]decan-4-amine dihydrochloride (54 mg, 0.22 mmol), and diisopropylethylamine (307 µL, 1.85 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-91**. HPLC-MS: [M+H]⁺=511.16/512.85. ¹H NMR (400 MHz, DMSO-*d*₆) δ 8.26 (d, *J* = 5.3 Hz, 1H), 7.89 (s, 1H), 7.68 (s, 1H), 7.50 (s, 1H), 6.55 (d, *J* = 5.3 Hz, 1H), 6.30 (s, 2H), 4.16 - 4.06 (m, 1H), 4.03 - 3.89 (m, 2H), 3.77 (s, 3H), 3.74 (d, *J* = 8.7 Hz, 1H), 3.55 (d, *J* = 8.7 Hz, 1H), 3.45 - 3.17 (m, 2H), 3.07 (d, *J* = 5.0 Hz, 1H), 1.86 - 1.38 (m, 4H), 1.12 (d, *J* = 6.4 Hz, 3H).

### Example 11

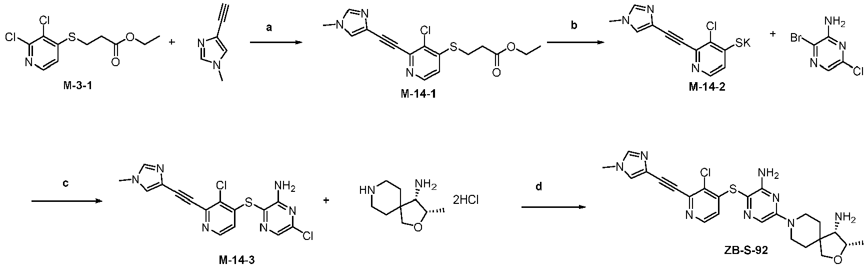

(a) The intermediate **M-3-1** (400 mg, 1.43 mmol), and 1-methyl-4-ethynyl-1H-imidazole (167 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-14-1.** HPLC-MS: [M+H]⁺=349.98/351.82. ¹H NMR (400 MHz, chloroform-*d*) δ 8.32 (d, *J* = 5.3 Hz, 1H), 7.49 (s, 1H), 7.27 (s, 1H), 7.01 (d, *J* = 5.3 Hz, 1H), 4.19 (q, *J* = 7.1 Hz, 2H), 3.72 (s, 3H), 3.25 (t, *J* = 7.4 Hz, 2H), 2.74 (t, *J* = 7.4 Hz, 2H), 1.28 (t, *J* = 7.1 Hz, 3H).
(b) The intermediate **M-14-1** (220 mg, 0.63 mmol) was dissolved in 15 mL of anhydrous tetrahydrofuran, and the atmosphere was replaced with argon. Potassium tert-butoxide (85 mg, 0.75 mmol) was added to the above system at room temperature, and the reaction was carried out for 1 h. The solvent was rotary evaporated off to obtain an intermediate **M-14-2,** which was directly used in the next step without purification.
(c) The intermediate **M-14-2** (165 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-14-3.** HPLC-MS: [M+H]⁺ =376.88/378.67/380.75.
(d) The intermediate **M-14-3** (70 mg, 0.19 mmol), (3S,4S)-3-methyl-2-oxa-8-azaspiro[4,5]decan-4-amine dihydrochloride (54 mg, 0.22 mmol), and diisopropylethylamine (307 µL, 1.85 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-92**. HPLC-MS: [M+H]⁺=511.16/512.76.

### Example 12

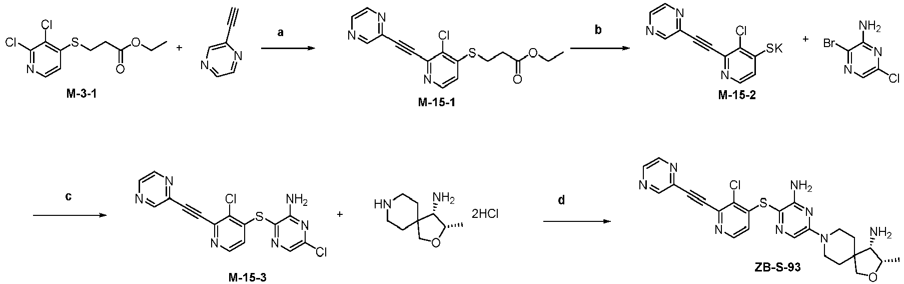

(a) The intermediate **M-3-1** (400 mg, 1.43 mmol), and 2-ethynylpyrazine (162 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-15-1.** HPLC-MS: [M+H]⁺ =347.90/349.79. ¹H NMR (400 MHz, chloroform-*d*) δ 8.86 (d, *J* = 1.5 Hz, 1H), 8.64 (dd, *J* = 2.5, 1.5 Hz, 1H), 8.55 (d, *J* = 2.5 Hz, 1H), 8.40 (d, *J* = 5.3 Hz, 1H), 7.12 (d, *J* = 5.3 Hz, 1H), 4.20 (q, *J* = 7.2 Hz, 2H), 3.28 (t, *J* = 7.4 Hz, 2H), 2.76 (t, *J* = 7.4 Hz, 2H), 1.29 (t, *J* = 7.2 Hz, 3H).
(b) The intermediate **M-15-1** (120 mg, 0.34 mmol) was dissolved in 15 mL of anhydrous tetrahydrofuran, and the atmosphere was replaced with argon. Potassium tert-butoxide (47 mg, 0.41mmol) was added to the above system at room temperature, and the reaction was carried out for 1 h. The solvent was rotary evaporated off to obtain an intermediate **M-15-2,** which was directly used in the next step without purification.
(c) The intermediate **M-15-2** (82 mg, 0.29 mmol), 2-amino-3-bromo-6-chloropyrazine (50 mg, 0.24 mmol), and potassium phosphate (101 mg, 0.48 mmol) were dispersed in 15 mL of 1,4-dioxane, and CuI (14 mg, 0.072 mmol) and phenanthroline (26 mg, 0.14 mmol) were added to the above system. The atmosphere was replaced with argon, and the reaction was carried out at 90 °C for 5 h. The reaction solution was concentrated, and the residue was separated by column chromatography to obtain an intermediate **M-15-3.** HPLC-MS: [M+H]⁺ =374.85/376.78/378.52.
(d) The intermediate **M-15-3** (40 mg, 0.11 mmol), (3S,4S)-3-methyl-2-oxa-8-azaspiro[4,5]decan-4-amine dihydrochloride (31 mg, 0.13 mmol), and diisopropylethylamine (176 µL, 1.06 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-93.** HPLC-MS: [M+H]⁺=509.13/510.92. ¹H NMR (400 MHz, methanol-*d*₄) δ 8.91 (d, *J* = 1.5 Hz, 1H), 8.69 (dd, *J* = 2.6, 1.5 Hz, 1H), 8.64 (d, *J* = 2.6 Hz, 1H), 8.22 (d, *J* = 5.3 Hz, 1H), 7.63 (s, 1H), 6.72 (d, *J* = 5.3 Hz, 1H), 4.30 - 4.20 (m, 1H), 4.19 - 4.06 (m, 2H), 3.90 (d, *J* = 8.7 Hz, 1H), 3.76 (d, *J* = 8.7 Hz, 1H), 3.41 - 3.19 (m, 2H), 3.11 (d, *J* = 4.8 Hz, 1H), 1.87 - 1.62 (m, 4H), 1.24 (d, *J* = 6.5 Hz, 3H).

### Example 13

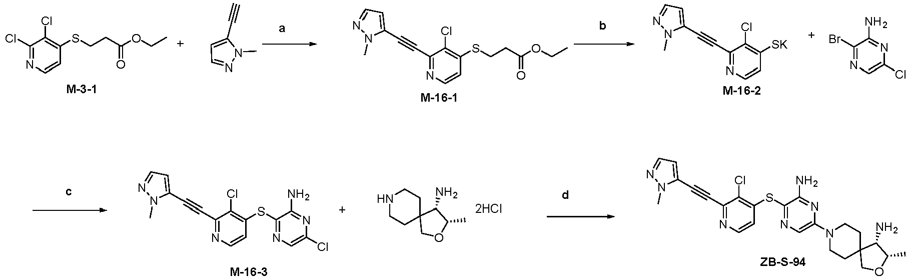

(a) The intermediate **M-3-1** (400 mg, 1.43 mmol), and 5-ethynyl-1-methyl-1H-pyrazole (167 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-16-1.** HPLC-MS: [M+H]⁺=349.93/351.77. ¹H NMR (400 MHz, chloroform-*d*) δ 8.37 (d, *J* = 5.3 Hz, 1H), 7.49 (d, *J* = 2.0 Hz, 1H), 7.08 (d, *J* = 5.3 Hz, 1H), 6.61 (d, *J* = 2.0 Hz, 1H), 4.20 (q, *J* = 7.2 Hz, 2H), 4.06 (s, 3H), 3.27 (t, *J* = 7.4 Hz, 2H), 2.76 (t, *J* = 7.4 Hz, 2H), 1.29 (t, *J* = 7.2 Hz, 3H).
(b) The intermediate **M-16-1** (140 mg, 0.40 mmol) was dissolved in 15 mL of anhydrous tetrahydrofuran, and the atmosphere was replaced with argon. Potassium tert-butoxide (54 mg, 0.48 mmol) was added to the above system at room temperature, and the reaction was carried out for 1 h. The solvent was rotary evaporated off to obtain an intermediate **M-16-2,** which was directly used in the next step without purification.
(c) The intermediate **M-16-2** (116 mg, 0.40 mmol), 2-amino-3-bromo-6-chloropyrazine (70 mg, 0.33 mmol), and potassium phosphate (142 mg, 0.67 mmol) were dispersed in 15 mL of 1,4-dioxane, and CuI (19 mg, 0.100 mmol) and phenanthroline (36 mg, 0.201 mmol) were added to the above system. The atmosphere was replaced with argon, and the reaction was carried out at 90 °C for 5 h. The reaction solution was concentrated, and the residue was separated by column chromatography to obtain an intermediate **M-16-3.** HPLC-MS: [M+H]⁺ =377.02/378.91/381.09.
(d) The intermediate **M-16-3** (65 mg, 0.17 mmol), (3S,4S)-3-methyl-2-oxa-8-azaspiro[4,5]decan-4-amine dihydrochloride (50 mg, 0.21 mmol), and diisopropylethylamine (285 µL, 1.72 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-94.** HPLC-MS: [M+H]⁺=511.16/513.00. ¹H NMR (400 MHz, DMSO-*d*₆) δ 8.29 (d, *J* = 5.3 Hz, 1H), 7.68 (s, 1H), 7.59 (d, *J* = 2.1 Hz, 1H), 6.79 (d, *J* = 2.1 Hz, 1H), 6.60 (d, *J* = 5.3 Hz, 1H), 6.30 (s, 2H), 4.15 - 4.05 (m, 1H), 4.00 (s, 3H), 3.99 - 3.87 (m, 2H), 3.73 (d, *J* = 8.6 Hz, 1H), 3.53 (d, *J* = 8.6 Hz, 1H), 3.43 - 3.20 (m, 2H), 3.02 (d, *J* = 5.1 Hz, 1H), 1.78 - 1.61 (m, 2H), 1.60 - 1.41 (m, 2H), 1.11 (d, *J* = 6.4 Hz, 3H).

### Example 14

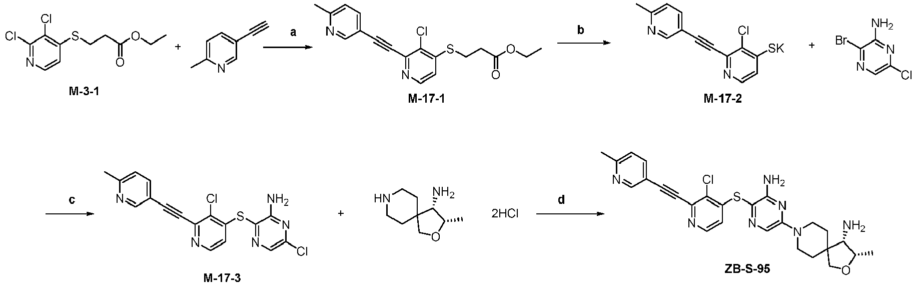

(a) The intermediate **M-3-1** (400 mg, 1.43 mmol), and 5-ethynyl-2-methyl-pyridine (184 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-17-1.** HPLC-MS: [M+H]⁺=361.20/363.12.
(b) The intermediate **M-17-1** (250 mg, 0.69 mmol) was dissolved in 15 mL of anhydrous tetrahydrofuran, and the atmosphere was replaced with argon. Potassium tert-butoxide (94 mg, 0.83 mmol) was added to the above system at room temperature, and the reaction was carried out for 1 h. The solvent was rotary evaporated off to obtain an intermediate **M-17-2,** which was directly used in the next step without purification.
(c) The intermediate **M-17-2** (172 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-17-3.** HPLC-MS: [M+H]⁺ =387.89/389.73/391.90.
(d) The intermediate **M-17-3** (70 mg, 0.17 mmol), (3S,4S)-3-methyl-2-oxa-8-azaspiro[4,5]decan-4-amine dihydrochloride (53 mg, 0.22 mmol), and diisopropylethylamine (299 µL, 1.80 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-95.** HPLC-MS: [M+H]⁺=522.24/524.13.

### Example 15

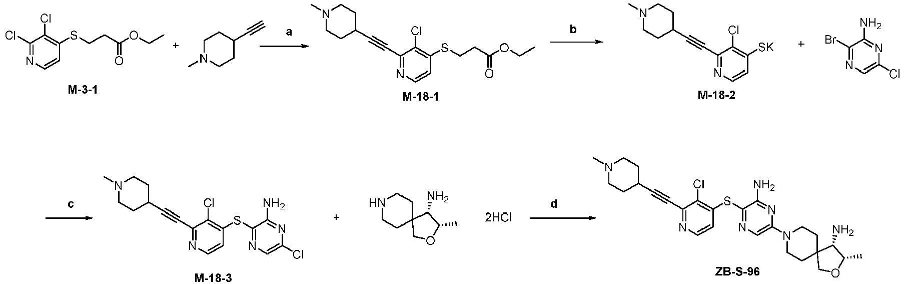

(a) The intermediate **M-3-1** (400 mg, 1.43 mmol), and 4-ethynyl-1-methyl-piperidine (194 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-18-1.** HPLC-MS: [M+H]⁺ =367.08/369.12.
(b) The intermediate **M-18-1** (250 mg, 0.68 mmol) was dissolved in 15 mL of anhydrous tetrahydrofuran, and the atmosphere was replaced with argon. Potassium tert-butoxide (92 mg, 0.82 mmol) was added to the above system at room temperature, and the reaction was carried out for 1 h. The solvent was rotary evaporated off to obtain an intermediate **M-18-2,** which was directly used in the next step without purification.
(c) The intermediate **M-18-2** (175 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-18-3.** HPLC-MS: [M+H]⁺ =394.12/396.07/398.13.
(d) The intermediate **M-18-3** (80 mg, 0.20 mmol), (3S,4S)-3-methyl-2-oxa-8-azaspiro[4,5]decan-4-amine dihydrochloride (59 mg, 0.24 mmol), and diisopropylethylamine (336 µL, 2.03 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-96.** HPLC-MS: [M+H]⁺=528.23/530.12.

### Example 16

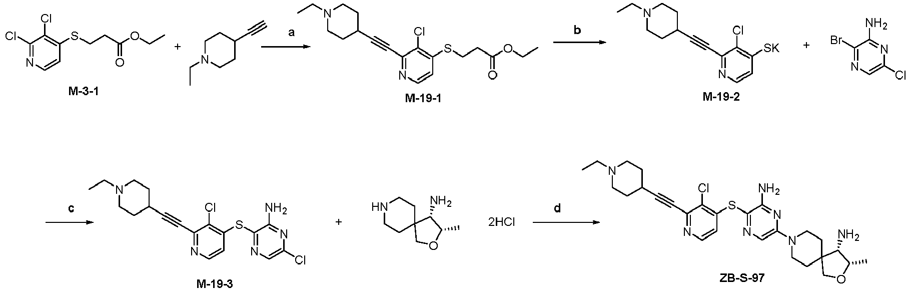

(a) The intermediate **M-3-1** (400 mg, 1.43 mmol), and 4-ethynyl-1-ethyl-piperidine (216 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-19-1.** HPLC-MS: [M+H]⁺=381.02/383.11.
(b) The intermediate **M-19-1** (300 mg, 0.79 mmol) was dissolved in 15 mL of anhydrous tetrahydrofuran, and the atmosphere was replaced with argon. Potassium tert-butoxide (107 mg, 0.95 mmol) was added to the above system at room temperature, and the reaction was carried out for 1 h. The solvent was rotary evaporated off to obtain an intermediate **M-19-2,** which was directly used in the next step without purification.
(c) The intermediate **M-19-2** (183 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-19-3.** HPLC-MS: [M+H]⁺ =408.02/409.93/412.04.
(d) The intermediate **M-19-3** (85 mg, 0.21 mmol), (3S,4S)-3-methyl-2-oxa-8-azaspiro[4,5]decan-4-amine dihydrochloride (61 mg, 0.25 mmol), and diisopropylethylamine (345 µL, 2.08 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-97.** HPLC-MS: [M+H]⁺=542.33/544.21.

### Example 17

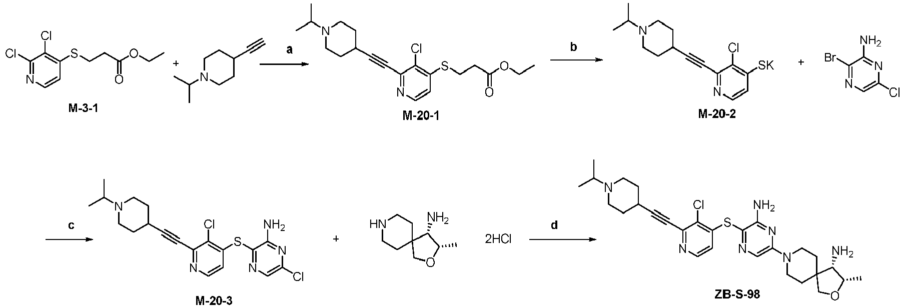

(a) The intermediate **M-3-1** (400 mg, 1.43 mmol), and 4-ethynyl-1-isopropyl-piperidine (238 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-20-1.** HPLC-MS: [M+H]⁺=395.21/397.12.
(b) The intermediate **M-20-1** (270 mg, 0.79 mmol) was dissolved in 15 mL of anhydrous tetrahydrofuran, and the atmosphere was replaced with argon. Potassium tert-butoxide (93 mg, 0.82 mmol) was added to the above system at room temperature, and the reaction was carried out for 1 h. The solvent was rotary evaporated off to obtain an intermediate **M-20-2,** which was directly used in the next step without purification.
(c) The intermediate **M-20-2** (191 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-20-3.** HPLC-MS: [M+H]⁺ =421.98/423.86/425.96.
(d) The intermediate **M-20-3** (90 mg, 0.21 mmol), (3S,4S)-3-methyl-2-oxa-8-azaspiro[4,5]decan-4-amine dihydrochloride (62 mg, 0.26 mmol), and diisopropylethylamine (353 µL, 2.13 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-98.** HPLC-MS: [M+H]⁺=556.14/558.09.

### Example 18

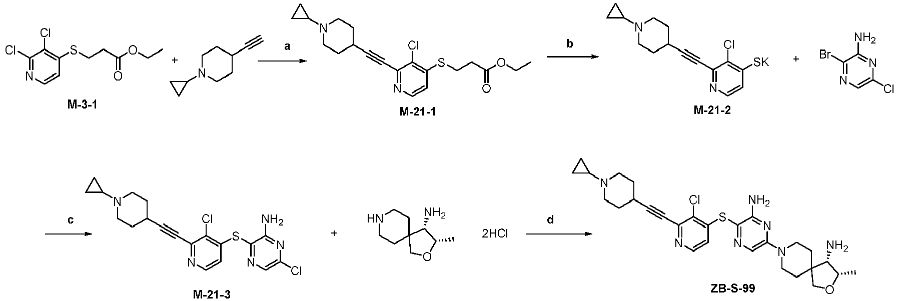

(a) The intermediate **M-3-1** (400 mg, 1.43 mmol), and 4-ethynyl-1-cyclopropyl-piperidine (235 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-21-1.** HPLC-MS: [M+H]⁺=393.14/395.25.
(b) The intermediate **M-21-1** (250 mg, 0.64 mmol) was dissolved in 15 mL of anhydrous tetrahydrofuran, and the atmosphere was replaced with argon. Potassium tert-butoxide (79 mg, 0.70 mmol) was added to the above system at room temperature, and the reaction was carried out for 1 h. The solvent was rotary evaporated off to obtain an intermediate **M-21-2,** which was directly used in the next step without purification.
(c) The intermediate **M-21-2** (190 mg, 0.57 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-21-3.** HPLC-MS: [M+H]⁺ =420.11/422.07/424.13.
(d) The intermediate **M-21-3** (75 mg, 0.18 mmol), (3S,4S)-3-methyl-2-oxa-8-azaspiro[4,5]decan-4-amine dihydrochloride (52 mg, 0.21 mmol), and diisopropylethylamine (295 µL, 1.78 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-99.** HPLC-MS: [M+H]⁺=554.19/556.05.

### Example 19

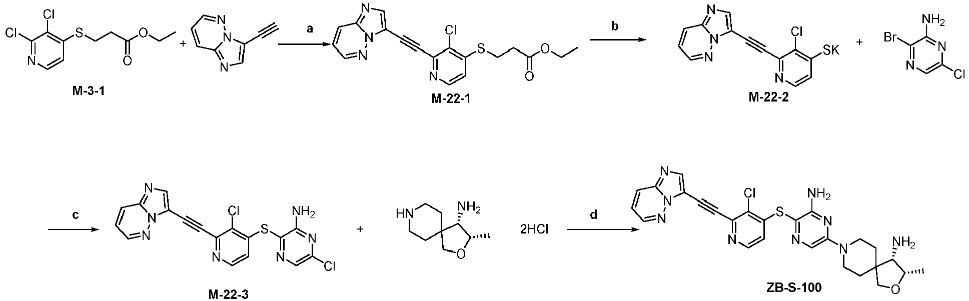

(a) The intermediate **M-3-1** (400 mg, 1.43 mmol), and 3-ethynylimidazole[1,2-b]pyridazine (225 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-22-1.** HPLC-MS: [M+H]⁺ =387.14/389.05. ¹H NMR (400 MHz, DMSO-*d*₆) δ 8.74 (dd, *J* = 4.4, 1.5 Hz, 1H), 8.44 (d, *J* = 5.3 Hz, 1H), 8.32 (s, 1H), 8.29 (dd, *J* = 9.2, 1.5 Hz, 1H), 7.48 (d, *J* = 5.3 Hz, 1H), 7.43 (dd, *J* = 9.2, 4.4 Hz, 2H), 4.11 (q, *J* = 7.1 Hz, 2H), 3.34 (d, *J* = 7.0 Hz, 2H), 2.78 (t, *J* = 7.0 Hz, 2H), 1.20 (t, *J* = 7.1 Hz, 3H).
(b) The intermediate **M-22-1** (250 mg, 0.65 mmol) was dissolved in 15 mL of anhydrous tetrahydrofuran, and the atmosphere was replaced with argon. Potassium tert-butoxide (87 mg, 0.78 mmol) was added to the above system at room temperature, and the reaction was carried out for 1 h. The solvent was rotary evaporated off to obtain an intermediate **M-22-2,** which was directly used in the next step without purification.
(c) The intermediate **M-22-2** (187 mg, 0.58 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-22-3.** HPLC-MS: [M+H]⁺ =414.01/416.12/418.06.
(d) The intermediate **M-22-3** (75 mg, 0.18 mmol), (3S,4S)-3-methyl-2-oxa-8-azaspiro[4,5]decan-4-amine dihydrochloride (52 mg, 0.21 mmol), and diisopropylethylamine (295 µL, 1.78 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-100.** ¹H NMR (400 MHz, DMSO-*d*₆) δ 8.74 (dd, *J* = 4.5, 1.6 Hz, 1H), 8.33 (s, 1H), 8.31 - 8.27 (m, 2H), 7.68 (s, 1H), 7.43 (dd, *J* = 9.2, 4.5 Hz, 1H), 6.59 (d, *J* = 5.3 Hz, 1H), 6.29 (s, 2H), 4.12 - 4.01 (m, 1H), 3.93 - 3.80 (m, 2H), 3.68 (d, *J* = 8.4 Hz, 1H), 3.49 (d, *J* = 8.4 Hz, 1H), 3.46 - 3.27 (m, 2H), 2.92 (d, *J* = 5.1 Hz, 1H), 1.79 - 1.68 (m, 1H), 1.68 - 1.57 (m, 1H), 1.57 - 1.42 (m, 2H), 1.08 (d, *J* = 6.3 Hz, 3H).

### Example 20

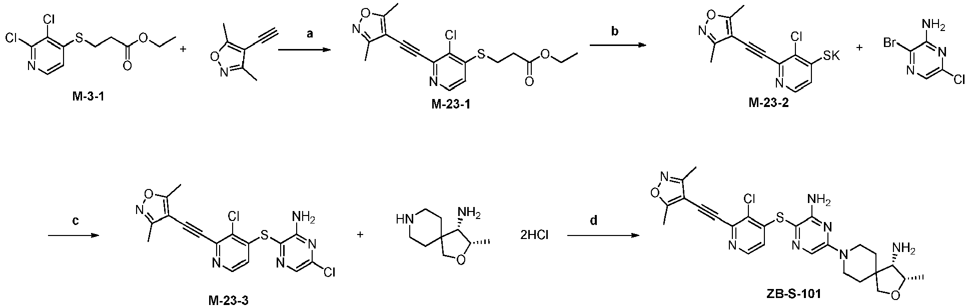

(a) The intermediate **M-3-1** (400 mg, 1.43 mmol), and 4-ethynyl-3,5-dimethylisoxazole (191 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-23-1.** HPLC-MS: [M+H]⁺=365.06/367.11.
(b) The intermediate **M-23-1** (250 mg, 0.69 mmol) was dissolved in 15 mL of anhydrous tetrahydrofuran, and the atmosphere was replaced with argon. Potassium tert-butoxide (92 mg, 0.82 mmol) was added to the above system at room temperature, and the reaction was carried out for 1 h. The solvent was rotary evaporated off to obtain an intermediate **M-23-2,** which was directly used in the next step without purification.
(c) The intermediate **M-23-2** (174 mg, 0.58 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-23-3.** HPLC-MS: [M+H]⁺ =371.05/393.11/395.04.
(d) The intermediate **M-23-3** (75 mg, 0.19 mmol), (3S,4S)-3-methyl-2-oxa-8-azaspiro[4,5]decan-4-amine dihydrochloride (52 mg, 0.23 mmol), and diisopropylethylamine (317 µL, 1.91 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-101.** HPLC-MS: [M+H]⁺=526.24/528.17. ¹H NMR (400 MHz, DMSO-*d*₆) δ 8.25 (d, *J* = 5.2 Hz, 1H), 7.67 (s, 1H), 6.57 (d, *J* = 5.3 Hz, 1H), 6.28 (s, 2H), 4.14 - 4.03 (m, 1H), 3.98 - 3.81 (m, 2H), 3.70 (d, *J* = 8.5 Hz, 1H), 3.51 (d, *J* = 8.5 Hz, 1H), 3.43 - 3.23 (m, 2H), 2.97 (d, *J* = 5.0 Hz, 1H), 2.56 (s, 3H), 2.33 (s, 3H), 1.79 - 1.59 (m, 2H), 1.59 - 1.42 (m, 2H), 1.10 (d, *J* = 6.4 Hz, 3H).

### Example 21

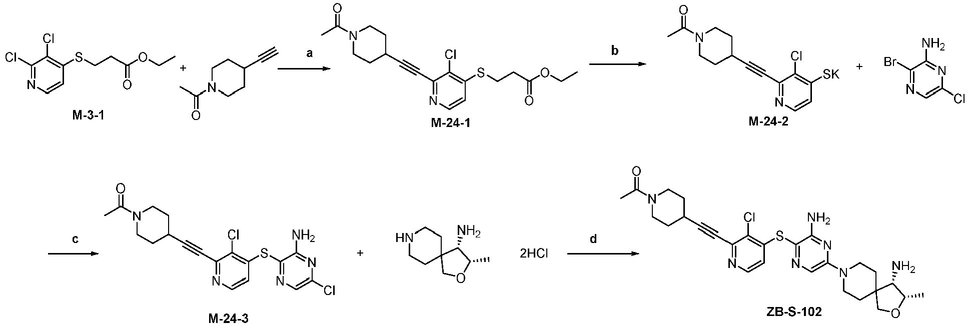

(a) The intermediate **M-3-1** (400 mg, 1.43 mmol), and N-acetyl-4-ethynylpiperidine (238 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-24-1.** HPLC-MS: [M+H]⁺=395.15/397.10. ¹H NMR (400 MHz, chloroform-*d*) δ 8.29 (d, *J* = 5.4 Hz, 1H), 7.02 (d, *J* = 5.3 Hz, 1H), 4.19 (q, *J* = 7.1 Hz, 2H), 3.86 - 3.78 (m, 1H), 3.76 - 3.67 (m, 1H), 3.66 - 3.57 (m, 1H), 3.47 - 3.36 (m, 1H), 3.24 (t, *J* = 7.4 Hz, 2H), 3.02 (tt, *J* = 7.2, 4.1 Hz, 1H), 2.73 (t, *J* = 7.4 Hz, 2H), 2.10 (s, 3H), 1.98 - 1.75 (m, 4H), 1.28 (t, *J* = 7.1 Hz, 3H).
(b) The intermediate **M-24-1** (250 mg, 0.69 mmol) was dissolved in 15 mL of anhydrous tetrahydrofuran, and the atmosphere was replaced with argon. Potassium tert-butoxide (85 mg, 0.76 mmol) was added to the above system at room temperature, and the reaction was carried out for 1 h. The solvent was rotary evaporated off to obtain an intermediate **M-24-2,** which was directly used in the next step without purification.
(c) The intermediate **M-24-2** (191 mg, 0.56 mmol), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-24-3.** HPLC-MS: [M+H]⁺ =422.07/4244.12/426.03.
(d) The intermediate **M-24-3** (80 mg, 0.19 mmol), (3S,4S)-3-methyl-2-oxa-8-azaspiro[4,5]decan-4-amine dihydrochloride (52 mg, 0.23 mmol), and diisopropylethylamine (317 µL, 1.91 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-102.** HPLC-MS: [M+H]⁺=556.31/558.25. ¹H NMR (400 MHz, DMSO-*d*₆) δ 8.18 (d, *J* = 5.2 Hz, 1H), 7.65 (s, 1H), 6.51 (d, *J* = 5.2 Hz, 1H), 6.24 (s, 2H), 4.13 - 4.00 (m, 1H), 3.92 - 3.75 (m, 3H), 3.70 - 3.61 (m, 2H), 3.48 (d, *J* = 8.5 Hz, 1H), 3.44 - 3.19 (m, 4H), 3.05 (tt, *J* = 8.1, 4.0 Hz, 1H), 2.90 (d, *J* = 5.1 Hz, 1H), 2.01 (s, 3H), 1.97 - 1.78 (m, 2H), 1.77 - 1.41 (m, 6H), 1.08 (d, *J* = 6.4 Hz, 3H).

### Example 22

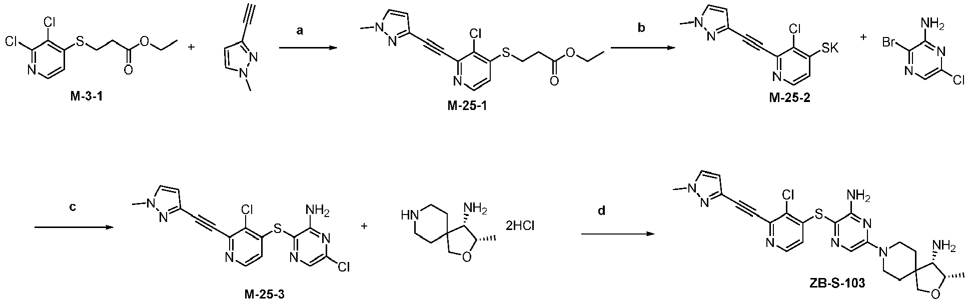

(a) The intermediate **M-3-1** (400 mg, 1.43 mmol), and 3-ethynyl-1-methyl-1H-pyrazole (167 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-25-1.** HPLC-MS: [M+H]⁺=349.94/351.79. ¹H NMR (400 MHz, chloroform-*d*) δ 8.33 (d, *J* = 5.3 Hz, 1H), 7.34 (d, *J* = 2.3 Hz, 1H), 7.03 (d, *J* = 5.3 Hz, 1H), 6.55 (d, *J* = 2.3 Hz, 1H), 4.18 (q, *J* = 7.1 Hz, 2H), 3.93 (s, 3H), 3.24 (t, *J* = 7.4 Hz, 2H), 2.73 (t, *J* = 7.4 Hz, 2H), 1.27 (t, *J* = 7.1 Hz, 3H).
(b) The intermediate **M-25-1** (140 mg, 0.40 mmol) was dissolved in 15 mL of anhydrous tetrahydrofuran, and the atmosphere was replaced with argon. Potassium tert-butoxide (54 mg, 0.48 mmol) was added to the above system at room temperature, and the reaction was carried out for 1 h. The solvent was rotary evaporated off to obtain an intermediate **M-25-2,** which was directly used in the next step without purification.
(c) The intermediate **M-25-2** (116 mg, 0.40 mmol), 2-amino-3-bromo-6-chloropyrazine (70 mg, 0.33 mmol), and potassium phosphate (142 mg, 0.67 mmol) were dispersed in 15 mL of 1,4-dioxane, and CuI (19 mg, 0.100 mmol) and phenanthroline (36 mg, 0.201 mmol) were added to the above system. The atmosphere was replaced with argon, and the reaction was carried out at 90 °C for 5 h. The reaction solution was concentrated, and the residue was separated by column chromatography to obtain an intermediate **M-25-3.** HPLC-MS: [M+H]⁺ =377.01/378.97/381.10.
(d) The intermediate **M-25-3** (65 mg, 0.17 mmol), (3S,4S)-3-methyl-2-oxa-8-azaspiro[4,5]decan-4-amine dihydrochloride (50 mg, 0.21 mmol), and diisopropylethylamine (285 µL, 1.72 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-103.** HPLC-MS: [M+H]⁺ =511.15/513.06. ¹H NMR (400 MHz, Methanol-*d*₄) δ 8.15 (d, *J* = 5.3 Hz, 1H), 7.67 (d, *J* = 2.3 Hz, 1H), 7.61 (s, 1H), 6.65 (d, *J* = 5.3 Hz, 1H), 6.60 (d, *J* = 2.3 Hz, 1H), 4.23 (qd, *J* = 6.4, 4.9 Hz, 1H), 4.12 - 3.99 (m, 2H), 3.94 (s, 3H), 3.86 (d, *J* = 8.7 Hz, 1H), 3.71 (d, *J* = 8.7 Hz, 1H), 3.44 - 3.23 (m, 2H), 3.01 (d, *J* = 5.0 Hz, 1H), 1.87 - 1.59 (m, 4H), 1.22 (d, *J* = 6.4 Hz, 3H).

### Example 23

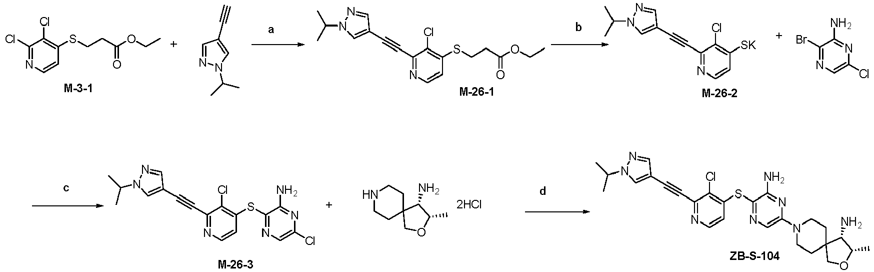

(a) The intermediate **M-3-1** (400 mg, 1.43 mmol), and 3-ethynyl-1-isopropyl-1H-pyrazole (211 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-26-1.** HPLC-MS: [M+H]⁺=378.12/380.06. ¹H NMR (400 MHz, chloroform-*d*) δ 8.31 (d, *J* = 5.3 Hz, 1H), 7.73 (s, 1H), 7.71 (s, 1H), 7.00 (d, *J* = 5.3 Hz, 1H), 4.50 (hept, *J* = 6.7 Hz, 1H), 4.19 (q, *J* = 7.1 Hz, 2H), 3.25 (t, *J* = 7.4 Hz, 2H), 2.74 (t, *J* = 7.4 Hz, 2H), 1.51 (d, *J* = 6.7 Hz, 6H), 1.27 (t, *J* = 7.1 Hz, 3H).
(b) The intermediate **M-26-1** (150 mg, 0.40 mmol) was dissolved in 15 mL of anhydrous tetrahydrofuran, and the atmosphere was replaced with argon. Potassium tert-butoxide (53 mg, 0.48 mmol) was added to the above system at room temperature, and the reaction was carried out for 1 h. The solvent was rotary evaporated off to obtain an intermediate **M-26-2,** which was directly used in the next step without purification.
(c) The intermediate **M-26-2** (127 mg, 0.40 mmol), 2-amino-3-bromo-6-chloropyrazine (70 mg, 0.33 mmol), and potassium phosphate (142 mg, 0.67 mmol) were dispersed in 15 mL of 1,4-dioxane, and CuI (19 mg, 0.100 mmol) and phenanthroline (36 mg, 0.201 mmol) were added to the above system. The atmosphere was replaced with argon, and the reaction was carried out at 90 °C for 5 h. The reaction solution was concentrated, and the residue was separated by column chromatography to obtain an intermediate **M-26-3.** HPLC-MS: [M+H]⁺ =405.06/407.12/409.02.
(d) The intermediate **M-26-3** (80 mg, 0.20 mmol), (3S,4S)-3-methyl-2-oxa-8-azaspiro[4,5]decan-4-amine dihydrochloride (48 mg, 0.24 mmol), and diisopropylethylamine (327 µL, 1.97 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-104.** HPLC-MS: [M+H]⁺=539.19/541.21. ¹H NMR (400 MHz, DMSO-*d*₆) δ 8.31 (s, 1H), 8.22 (d, *J* = 5.3 Hz, 1H), 7.80 (s, 1H), 7.66 (s, 1H), 6.51 (d, *J* = 5.3 Hz, 1H), 6.27 (s, 2H), 4.54 (hept, *J* = 6.7 Hz, 1H), 4.12 - 3.99 (m, 1H), 3.92 - 3.76 (m, 2H), 3.67 (d, *J* = 8.4 Hz, 1H), 3.48 (d, *J* = 8.4 Hz, 1H), 3.45 - 3.21 (m, 2H), 2.90 (d, *J* = 5.1 Hz, 1H), 1.79 - 1.67 (m, 1H), 1.67 - 1.57 (m, 1H), 1.56 - 1.45 (m, 2H), 1.43 (d, *J* = 6.7 Hz, 6H), 1.08 (d, *J* = 6.4 Hz, 3H).

### Example 24

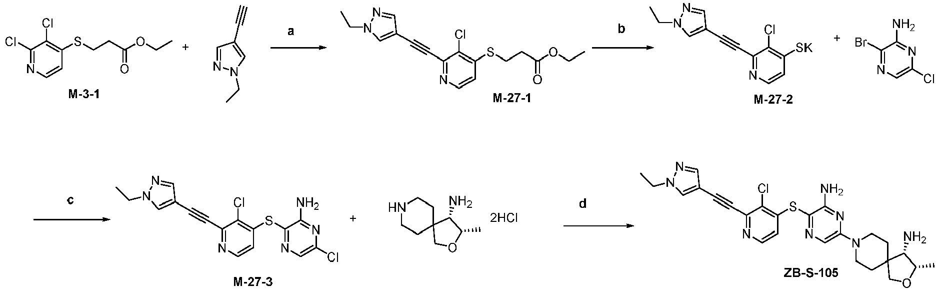

(a) The intermediate **M-3-1** (400 mg, 1.43 mmol), and 3-ethynyl-1-ethyl-1H-pyrazole (189 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-27-1.** HPLC-MS: [M+H]⁺=364.01/366.12. ¹H NMR (400 MHz, chloroform-d) δ 8.31 (d, *J* = 5.3 Hz, 1H), 7.72 (s, 1H), 7.68 (s, 1H), 7.00 (d, *J* = 5.3 Hz, 1H), 4.22 - 4.13 (m, 4H), 3.24 (t, *J* = 7.4 Hz, 2H), 2.73 (t, *J* = 7.4 Hz, 2H), 1.49 (t, *J* = 7.3 Hz, 3H), 1.27 (t, *J* = 7.1 Hz, 3H).
(b) The intermediate **M-27-1** (150 mg, 0.41 mmol) was dissolved in 15 mL of anhydrous tetrahydrofuran, and the atmosphere was replaced with argon. Potassium tert-butoxide (56 mg, 0.49 mmol) was added to the above system at room temperature, and the reaction was carried out for 1 h. The solvent was rotary evaporated off to obtain an intermediate **M-27-2,** which was directly used in the next step without purification.
(c) The intermediate **M-27-2** (127 mg, 0.40 mmol), 2-amino-3-bromo-6-chloropyrazine (70 mg, 0.33 mmol), and potassium phosphate (142 mg, 0.67 mmol) were dispersed in 15 mL of 1,4-dioxane, and CuI (19 mg, 0.100 mmol) and phenanthroline (36 mg, 0.201 mmol) were added to the above system. The atmosphere was replaced with argon, and the reaction was carried out at 90 °C for 5 h. The reaction solution was concentrated, and the residue was separated by column chromatography to obtain an intermediate **M-27-3.** HPLC-MS: [M+H]⁺ =391.03/393.12/395.03.
(d) The intermediate **M-27-3** (80 mg, 0.20 mmol), (3S,4S)-3-methyl-2-oxa-8-azaspiro[4,5]decan-4-amine dihydrochloride (50 mg, 0.25 mmol), and diisopropylethylamine (339 µL, 2.04 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-105.** HPLC-MS: [M+H]⁺=525.31/527.22. ¹H NMR (400 MHz, DMSO-*d*₆) δ 8.28 (s, 1H), 8.22 (d, *J* = 5.3 Hz, 1H), 7.80 (s, 1H), 7.66 (s, 1H), 6.51 (d, *J* = 5.3 Hz, 1H), 6.27 (s, 2H), 4.17 (q, *J* = 7.3 Hz, 2H), 4.10 - 4.02 (m, 1H), 3.92 - 3.76 (m, 2H), 3.67 (d, *J* = 8.4 Hz, 1H), 3.48 (d, *J* = 8.4 Hz, 1H), 3.45 - 3.26 (m, 2H), 2.90 (d, *J* = 5.1 Hz, 1H), 1.77 - 1.67 (m, 1H), 1.66 - 1.56 (m, 1H), 1.56 - 1.41 (m, 2H), 1.39 (t, *J* = 7.3 Hz, 3H), 1.08 (d, *J* = 6.4 Hz, 3H).

### Example 25

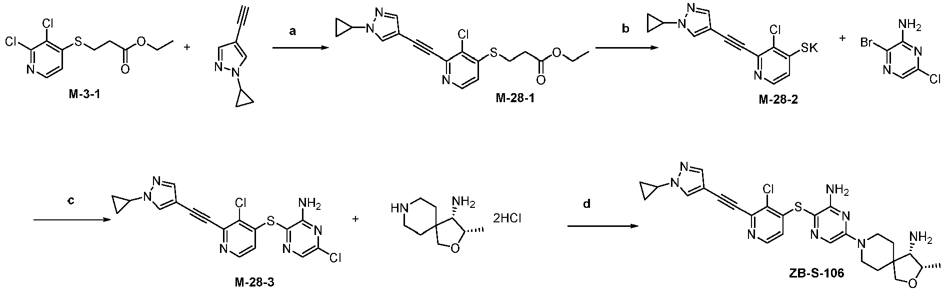

(a) The intermediate **M-3-1** (400 mg, 1.43 mmol), and 3-ethynyl-1-cyclopropyl-1H-pyrazole (208 mg, 1.57 mmol) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-28-1.** HPLC-MS: [M+H]⁺ =376.12/378.06. ¹H NMR (400 MHz, chloroform-d) δ 8.31 (d, *J* = 5.3 Hz, 1H), 7.73 (s, 1H), 7.69 (s, 1H), 7.00 (d, *J* = 5.3 Hz, 1H), 4.18 (q, *J* = 7.1 Hz, 2H), 3.60 (tt, *J* = 7.4, 3.8 Hz, 1H), 3.24 (t, *J* = 7.4 Hz, 2H), 2.73 (t, *J* = 7.4 Hz, 2H), 1.27 (t, *J* = 7.1 Hz, 3H), 1.16 - 1.09 (m, 2H), 1.08 - 1.01 (m, 2H).
(b) The intermediate **M-28-1** (150 mg, 0.40 mmol) was dissolved in 15 mL of anhydrous tetrahydrofuran, and the atmosphere was replaced with argon. Potassium tert-butoxide (54 mg, 0.48 mmol) was added to the above system at room temperature, and the reaction was carried out for 1 h. The solvent was rotary evaporated off to obtain an intermediate **M-28-2,** which was directly used in the next step without purification.
(c) The intermediate **M-28-2** (126 mg, 0.40 mmol), 2-amino-3-bromo-6-chloropyrazine (70 mg, 0.33 mmol), and potassium phosphate (142 mg, 0.67 mmol) were dispersed in 15 mL of 1,4-dioxane, and CuI (19 mg, 0.100 mmol) and phenanthroline (36 mg, 0.201 mmol) were added to the above system. The atmosphere was replaced with argon, and the reaction was carried out at 90 °C for 5 h. The reaction solution was concentrated, and the residue was separated by column chromatography to obtain an intermediate **M-28-3.** HPLC-MS: [M+H]⁺ =403.05/405.11/407.09.
(d) The intermediate **M-28-3** (80 mg, 0.20 mmol), and (3S,4S)-3-methyl-2-oxa-8-azaspiro[4,5]decan-4-amine dihydrochloride (50 mg, 0.25 mmol), and diisopropylethylamine (329 µL, 2.04 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-106.** HPLC-MS: [M+H]⁺=537.21/539.16. ¹H NMR (400 MHz, DMSO-*d*₆) δ 8.32 (s, 1H), 8.22 (d, *J* = 5.3 Hz, 1H), 7.79 (s, 1H), 7.66 (s, 1H), 6.52 (d, *J* = 5.3 Hz, 1H), 6.27 (s, 2H), 4.13 - 4.00 (m, 1H), 3.91 - 3.74 (m, 3H), 3.66 (d, *J* = 8.4 Hz, 1H), 3.48 (d, *J* = 8.4 Hz, 1H), 3.45 - 3.26 (m, 2H), 2.89 (d, *J* = 5.1 Hz, 1H), 1.79 - 1.68 (m, 1H), 1.68 - 1.56 (m, 1H), 1.56 - 1.41 (m, 2H), 1.12 - 1.04 (m, 5H), 1.02 - 0.95 (m, 2H).

### Example 26

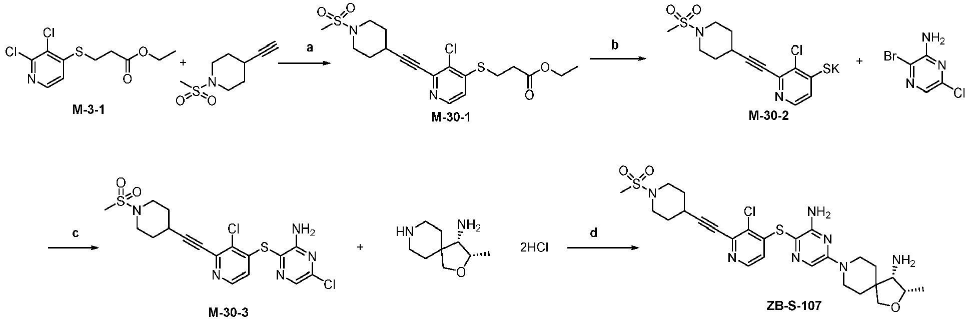

(a) The intermediate **M-3-1** (400 mg, 1.43 mmol), and N-mesyl-4-ethynylpiperidine (260 mg) were dissolved in 30 mL of a mixed solvent of 1,4-dioxane/diisopropylamine (20 mL/10 mL). Pd(OAc)₂ (32 mg, 0.14 mmol), Xphos (136 mg, 0.29 mmol), and CuI (54 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-30-1**. HPLC-MS: [M+H]⁺ =431.1.
(b) The intermediate **M-30-1** (250 mg) was dissolved in 15 mL of anhydrous tetrahydrofuran, and the atmosphere was replaced with argon. Potassium tert-butoxide (85 mg, 0.76 mmol) was added to the above system at room temperature, and the reaction was carried out for 1 h. The solvent was rotary evaporated off to obtain an intermediate **M-30-2**, which was directly used in the next step without purification.
(c) The intermediate **M-30-2** (190 mg), 2-amino-3-bromo-6-chloropyrazine (100 mg, 0.48 mmol), and potassium phosphate (203 mg, 0.96 mmol) were dispersed in 15 mL of 1,4-dioxane, and CuI (27 mg, 0.14 mmol) and phenanthroline (52 mg, 0.29 mmol) were added to the above system. The atmosphere was replaced with argon, and the reaction was carried out at 90 °C for 5 h. The reaction solution was concentrated, and the residue was separated by column chromatography to obtain an intermediate **M-30-3**.
(d) The intermediate **M-30-3** (80 mg), and (3S,4S)-3-methyl-2-oxa-8-azaspiro[4,5]decan-4-amine dihydrochloride (52 mg, 0.23 mmol), and diisopropylethylamine (317 µL, 1.91 mmol) were dissolved in 10 mL of DMSO. The atmosphere was replaced with argon, and the reaction was carried out at 120 °C for 10 h. The reaction solution was cooled to room temperature. 30 mL of saturated brine was added, and the mixture was extracted three times with ethyl acetate (20 mL*3), and the ethyl acetate layers were combined. The organic phase was washed with saturated brine, dried over anhydrous sodium sulfate and concentrated. The residue was separated by column chromatography to obtain **ZB-S-107**. HPLC-MS: [M+H]⁺=592.2.

### Bioactivity assaies

Example 27: Phosphatase activity inhibition assay (determination of IC₅₀):
(a) The positive control SHP099 was prepared according to the reported literature (J Med Chem 2016, 59 (17), 7773-82); and the positive control TNO155 was prepared according to the reported literature (J Med Chem 2020, 63 (22), 13578-13594).
(b) 6,8-difluoro-4-methylumbelliferyl phosphate (DiFMUP) was used as the reaction substrate, and a solution of full-length SHP2 (Met1-Arg 593) (diluted to 0.5 nM in the reaction solution) and the peptide H₂N-LN(pY)IDLDLV(dPEG8)LST(pY)ASINFQK-amide were incubated together in the reaction solution (60 mM 3,3-4-(2-hydroxyethyl)-1-piperazinethanesulfonic acid (HEPES), pH=7.2, 75 mM NaCl, 75 mM KCl, 1 mM EDTA, 0.05% Tween-20, 5 mM dithiothreitol (DTT)) for 30 to 60 minutes to activate the SHP2, and DMSO (1% (V/V) or the compound (10 µM to 0.1 nM) was added to the mixture, and the incubation continued at room temperature for 20 minutes. DiFMUP (25 µM, the total volume of the reaction solution was 50 µL) was added, and the reaction started. The fluorescence intensity of the reaction solution was detected by using the Envision multifunctional microplate reader (PerkinElmer) (Excitation light 355 nm, emission light 460 nm). Three duplicate wells were set for each dose. The fluorescence value of the control well (DMSO) was set to 100%. The IC₅₀ of the inhibitory activity of the compounds of the present invention against the SHP2 is shown in Table 1.

**Table 1: SHP2 activity inhibition experiments**

| Compound | IC₅₀ (nM) |
|---|---|
| SHP099 | 155.4±19.7 |
| TNO155 | 9.0±1.3 |
| ZB-S-82 | 9.8±0.7 |
| ZB-S-83 | 7.2±0.4 |
| ZB-S-84 | 4.1±0.0 |
| ZB-S-85 | 5.5±0.4 |
| ZB-S-86 | 9.8±1.6 |
| ZB-S-87 | 5.8±0.6 |
| ZB-S-88 | 10.8±0.5 |
| ZB-S-89 | 20.7±1.0 |
| ZB-S-90 | 63.8±4.8 |
| ZB-S-91 | 15.4 |
| ZB-S-92 | 10.0 |
| ZB-S-93 | 17.5 |
| ZB-S-94 | 14.8 |
| ZB-S-100 | 23.0 |
| ZB-S-101 | 27.5 |
| ZB-S-102 | 22.6 |
| ZB-S-103 | 7.1 |
| ZB-S-104 | 4.1 |
| ZB-S-105 | 4.7 |
| ZB-S-106 | 4.3 |

Example 28: Cell proliferation inhibition assay (determination of IC₅₀):
(a) SHP099 and TNO155 were used as positive controls.
(b) MV4-11 cells (10,000 cells/well) were seeded in a 96-well culture plate with 100 µL/well of the culture medium (IMDM supplemented with 10% fetal bovine serum (FBS), from Gibco company), and cultured for 6 hours. The compounds formulated in different concentrations of the present invention were added. On the third day, 20 µL of MTS reagent (purchased from Promega Company) was added to each well, and the absorbance value was detected according to the reagent instructions (Promega Company). The IC₅₀ of the inhibitory activity of the compounds of the present invention against the MV4-11 cell proliferation is shown in Table 2.

**Table 2: MV4-11 cell proliferation inhibition experiments**

| Compound | IC₅₀ (nM) |
|---|---|
| SHP099 | 882.9±104.8 |
| TNO155 | 118.0 ± 5.8 |
| ZB-S-82 | 10.3±0.6 |
| ZB-S-83 | 27.0±4.5 |
| ZB-S-84 | 14.0±0.4 |
| ZB-S-85 | 8.3±0.7 |
| ZB-S-86 | 20.0±2.4 |
| ZB-S-87 | 14.8±2.0 |
| ZB-S-88 | 3.1±0.4 |
| ZB-S-89 | 28.6±0.6 |
| ZB-S-90 | 94.9±2.9 |
| ZB-S-92 | <20 |
| ZB-S-93 | <20 |
| ZB-S-95 | <20 |
| ZB-S-96 | <20 |
| ZB-S-91 | 21.8 |
| ZB-S-100 | 23.5 |
| ZB-S-101 | 80.1 |
| ZB-S-102 | 22.8 |
| ZB-S-103 | 2.3 |
| ZB-S-104 | 7.4 |
| ZB-S-105 | 3.1 |
| ZB-S-106 | 4.1 |

From the above results, it can be known that most of the compounds according to the present invention are 40 to 100 times more potent than the positive compound SHP099, and 5-40 times more potent than the clinical compound TNO155.

### Example 29: Cell protein kinase (p-ERK) phosphorylation inhibition experiment

The inhibition of the phosphorylation level of intracellular protein kinase (ERK) by a compound was detected by AlphaLISA method. The first step was to treat the cells with the compound. The compound to be tested was first diluted 5-fold with 100% DMSO, and a gradient including a total of 9 different concentrations was set; then MIAPaca-2 cells were inoculated into a 96-well plate at a density of 40,000 cells per well, with a volume of 80 µL per well; then 20 µL of DMSO or the compound to be tested with different concentrations was added to each well, with 2 replicates for each concentration, and the final concentration of DMSO was controlled at 0.5%. The second step was to lyse the cells. After the cells were treated for 2 hours, the culture medium was removed, and the cells were washed three times with a HBSS solution. 50 µl of a freshly prepared lysis buffer was added to each well, shaken, and left at room temperature for 10 minutes. The third step was to detect the phosphorylated extracellular signal-regulated kinase (p-ERK) using a AphaLISA SureFire ^{®} UltraTM p-ERK 1/2 (Thr202/Tyr204) kit (Perkin Elmer, ALSU-PERK-A500). 10 µL of the above lysate was put into a 384-well plate (Perkin Elmer, 6005350), and the phosphorylation level of the extracellular signal-regulated kinase in the sample was detected according to the manufacturer's instructions. Signals were read using the AlphaScreen detector on an Envision multifunctional microplate reader (PerkinElmer). The inhibition (%) was calculated by the following equation: Inhibition (%) = (1 - p-ERK signal of compound-treated cells / p-ERK signal of DMSO-treated cells)* 100

Results: IC₅₀ values of some compounds of the present invention were shown in the table below.

| Compound | IC₅₀ (nM) |
|---|---|
| TNO155 | 30 |
| ZB-S-85 | 3.2 |
| ZB-S-88 | 1.7 |

It can be known from the results that the inhibitory activity of the selected compounds against the phosphorylated cellular protein kinase (p-ERK) was significantly stronger than that of the positive compound TNO155.

## Claims

1. A compound represented by formula I, or a pharmaceutically acceptable salt, enantiomer, diastereomer, atropisomer, racemate, polymorph, solvate or isotopically labeled compound thereof: 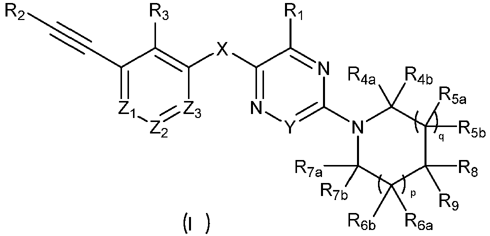 wherein
R₁ is selected from hydrogen, -NH₂, -OH, -CN, halogen, C₁-C₄ alkyl, C₁-C₄ haloalkyl, and C₃-C₆ cycloalkyl;
R₂ is selected from hydrogen, C₁-C₁₀ alkyl, C₁-C₁₀ haloalkyl, C₃-C₈ cycloalkyl, 3- to 10-membered heterocyclyl, C₆-C₁₀ aryl, and 5- to 10-membered heteroaryl; the C₁-C₁₀ alkyl, C₁-C₁₀ haloalkyl, C₃-C₈ cycloalkyl, 3- to 10-membered heterocycloalkyl, C₆-C₁₀ aryl, and 5- to 10-membered heteroaryl are optionally further substituted by one to finve substituents selected from -NR₁₀R₁₁, -OR₁₀, -CN, halogen, C₁-C₁₀ alkyl, C₁-C₁₀ haloalkyl, C₃-C₈ cycloalkyl, 3- to 10-membered heterocyclyl, -C(=O)R₁₀, -C(=O)OR₁₀, -C(=O)NR₁₀R₁₁, -S(=O)₂R₁₀, -S(=O)₂NR₁₀R₁₁, and -NR₁₀C(=O)R₁₁; wherein, R₁₀ and R₁₁ are independently selected from hydrogen, C₁-C₄ alkyl, and C₃-C₇ cycloalkyl;
R₃ is selected from hydrogen, halogen, C₁-C₄ alkyl, C₃-C₈ cycloalkyl, C₁-C₄ alkoxy, and C₁-C₄ haloalkyl;
R₄ₐ, R_{4b}, R₅ₐ, R_{5b}, R₆ₐ, R_{6b}, R₇ₐ and R_{7b} are each independently selected from hydrogen, -NH₂, -OH, halogen, carbonyl, C₁-C₄ alkyl, C₁-C₄ alkoxy, C₃-C₈ cycloalkyl and C₁-C₄ alkylamino;
R₈ and R₉ are each independently selected from -NH₂, -OH, -CN, -COOH, halogen, C₁-C₄ alkyl, C₁-C₄ alkoxy, and C₁-C₄ alkylamino; wherein the C₁-C₄ alkyl is optionally substituted by one to three substituents selected from -NH₂, -OH, and -CN;
alternatively, any two groups selected from R₄ₐ, R_{4b}, R₅ₐ, R_{5b}, R₆ₐ, R_{6b}, R₇ₐ, R_{7b} and R₈ are combined with the carbon atoms connected thereto to form a 5- to 6-membered saturated or unsaturated ring;
alternatively, R₈ and R₉ form a 3- to 7-membered saturated or unsaturated ring together with the carbon atom connected thereto, and the 3- to 7-membered saturated or unsaturated ring optionally contains one to three heteroatoms or groups independently selected from N, O, C(=O), and S(=O)ₘ, wherein m=1 or 2; the saturated or unsaturated ring is optionally substituted by one to three substituents selected from -NH₂, -OH, -CN, halogen, C₁-C₄ alkyl, C₁-C₄ alkoxy, and C₁-C₄ alkylamino;
p is 0 or 1;
q is 0 or 1;
X is S or absent;
Yis CR₁₂ or N;
R₁₂ is selected from hydrogen, -NH₂, -CN, halogen, C₁-C₄ alkyl, -OR₁₃, C₃-C₈ cycloalkyl, 3- to 10-membered heterocyclyl, -C(=O)R₁₃, -C(=O)OR₁₃, -C(=O)NR₁₃R₁₄, -S(=O)₂NR₁₃R₁₄ and -S(=O)₂R₁₃, wherein R₁₃ and R₁₄ are each independently selected from hydrogen, C₁-C₄ alkyl and C₃-C₆ cycloalkyl;
Z₁ is N or CR₁₅;
Z₂ is N or CR₁₆;
Z₃ is N or CR₁₇;
R₁₅, R₁₆, R₁₇ are each independently selected from hydrogen, -CN, halogen, -C(=O)R₁₈, -C(=O)OR₁₈, -C(=O)NR₁₈R₁₉, -NR₁₈R₁₉, -NR₁₈C(=O)R₁₉, -NR₁₈C(=O)OR₁₉, -OC(=O)R₁₈, -OC(=O)NR₁₈R₁₉, -OR₁₈, -S(=O)₂NR₁₈R₁₉, -S(=O)₂R₁₈, C₁-C₄ alkyl, C₃-C₈ cycloalkyl, 3- to 10-membered heterocyclyl, C₆-C₁₀ aryl and 5- to 10-membered heteroaryl; R₁₈ and R₁₉ are each independently selected from hydrogen, C₁-C₄ alkyl and C₃-C₈ cycloalkyl; the C₁-C₄ alkyl, C₃-C₈ cycloalkyl in R₁₅ to R₁₉, the 3- to 10-membered heterocyclyl, C₆-C₁₀ aryl, 5- to 10-membered heteroaryl in R₁₅ to R₁₇ are optionally further substituted by one to three substituents independently selected from -NH₂, -OH, -CN, halogen, C₁-C₄ alkyl, C₃-C₆ cycloalkyl and C₁-C₄ alkoxy.

2. The compound, or a pharmaceutically acceptable salt, enantiomer, diastereomer, atropisomer, racemate, polymorph, solvate or isotopically labeled compound thereof according to claim 1, wherein the compound represented by formula I is selected from the compounds represented by formula Ia: 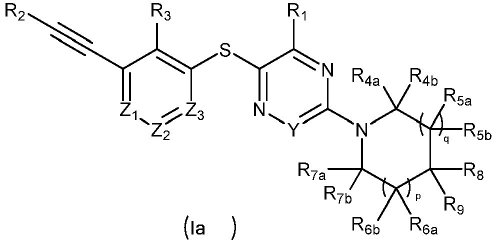 wherein
R₁ is selected from hydrogen, -NH₂, C₁-C₄ alkyl and C₁-C₄ haloalkyl;
R₂ is selected from hydrogen, C₁-C₁₀ alkyl, C₁-C₁₀ haloalkyl, C₃-C₈ cycloalkyl, 3- to 10-membered heterocyclyl, C₆-C₁₀ aryl, and 5- to 10-membered heteroaryl; the C₁-C₁₀ alkyl, C₁-C₁₀ haloalkyl, C₃-C₈ cycloalkyl, 3- to 10-membered heterocycloalkyl, C₆-C₁₀ aryl, and 5- to 10-membered heteroaryl are optionally further substituted by one to five substituents selected from -NR₁₀R₁₁, -OR₁₀, -CN, halogen, C₁-C₁₀ alkyl, C₁-C₁₀ haloalkyl, C₃-C₈ cycloalkyl, 3- to 10-membered heterocyclyl, -C(=O)R₁₀, -C(=O)OR₁₀, -C(=O)NR₁₀R₁₁, -S(=O)₂R₁₀, -S(=O)₂NR₁₀R₁₁, and -NR₁₀C(=O)R₁₁; wherein, R₁₀ and R₁₁ are independently selected from hydrogen, C₁-C₄ alkyl, and C₃-C₇ cycloalkyl;
R₃ is selected from hydrogen, C₁-C₄ alkyl and halogen;
R₄ₐ, R_{4b}, R₅ₐ, R_{5b}, R₆ₐ, R_{6b}, R₇ₐ and R_{7b} are each independently selected from hydrogen, -NH₂, -OH, halogen, carbonyl, C₁-C₄ alkyl, C₁-C₄ alkoxy, C₃-C₈ cycloalkyl and C₁-C₄ alkylamino;
R₈ and R₉ are each independently selected from -NH₂, -OH, -CN, -COOH, halogen, C₁-C₄ alkyl, C₁-C₄ alkoxy, and C₁-C₄ alkylamino; wherein the C₁-C₄ alkyl is optionally substituted by one to three substituents selected from -NH₂, -OH, and -CN;
alternatively, R₈ and R₉ form a 3- to 7-membered saturated or unsaturated ring together with the carbon atom connected thereto, and the 3- to 7-membered saturated or unsaturated ring optionally contains one to three heteroatoms or groups independently selected from N, O, C(=O), and S(=O)ₘ, wherein m=1 or 2; the saturated or unsaturated ring is optionally substituted by one to three substituents selected from -NH₂, -OH, -CN, halogen, C₁-C₄ alkyl, C₁-C₄ alkoxy, and C₁-C₄ alkylamino;
p is 0 or 1;
q is 0 or 1;
Y is CR₁₂ or N;
R₁₂ is selected from hydrogen, -NH₂, -CN, halogen, C₁-C₄ alkyl, -OR₁₃, C₃-C₈ cycloalkyl, 3- to 10-membered heterocyclyl, -C(=O)R₁₃, -C(=O)OR₁₃, -C(=O)NR₁₃R₁₄, -S(=O)₂NR₁₃R₁₄ and -S(=O)₂R₁₃, wherein R₁₃ and R₁₄ are each independently selected from hydrogen, C₁-C₄ alkyl and C₃-C₆ cycloalkyl;
Z₁ is N or CR₁₅;
Z₂ is N or CR₁₆;
Z₃ is N or CR₁₇;
R₁₅, R₁₆, R₁₇ are each independently selected from hydrogen, -CN, halogen, C₁-C₄ alkyl, C₃-C₈ cycloalkyl, 3- to 10-membered heterocyclyl, C₆-C₁₀ aryl and 5- to 10-membered heteroaryl; the C₁-C₄ alkyl, C₃-C₈ cycloalkyl, 3- to 10-membered heterocyclyl, C₆-C₁₀ aryl, 5-to 10-membered heteroaryl are optionally further substituted by one to three substituents independently selected from -NH₂, -OH, -CN, halogen, C₁-C₄ alkyl, C₃-C₆ cycloalkyl and C₁-C₄ alkoxy.

3. The compound, or a pharmaceutically acceptable salt, enantiomer, diastereomer, atropisomer, racemate, polymorph, solvate or isotopically labeled compound thereof according to claim 1, wherein the compound represented by formula I is selected from the compounds represented by formula Ib: 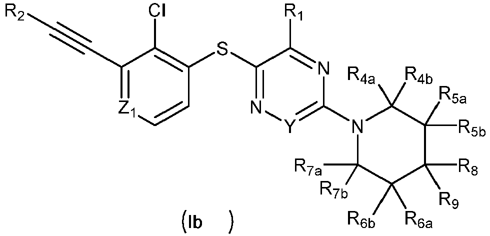 wherein
R₁ is selected from hydrogen, -NH₂, methyl and trifluoromethyl;
R₂ is selected from hydrogen, C₁-C₁₀ alkyl, C₁-C₁₀ haloalkyl, C₃-C₈ cycloalkyl, 3- to 10-membered heterocyclyl, C₆-C₁₀ aryl, and 5- to 10-membered heteroaryl; the C₁-C₁₀ alkyl, C₁-C₁₀ haloalkyl, C₃-C₈ cycloalkyl, 3- to 10-membered heterocycloalkyl, C₆-C₁₀ aryl, and 5- to 10-membered heteroaryl are optionally further substituted by one to five substituents selected from -NR₁₀R₁₁, -OR₁₀, -CN, halogen, C₁-C₁₀ alkyl, C₁-C₁₀ haloalkyl, C₃-C₈ cycloalkyl, 3- to 10-membered heterocyclyl, -C(=O)R₁₀, -C(=O)OR₁₀, -C(=O)NR₁₀R₁₁, -S(=O)₂R₁₀, -S(=O)₂NR₁₀R₁₁, and -NR₁₀C(=O)R₁₁; wherein, R₁₀ and R₁₁ are independently selected from hydrogen and C₁-C₄ alkyl;
R₄ₐ, R_{4b}, R₅ₐ, R_{5b}, R₆ₐ, R_{6b}, R₇ₐ and R_{7b} are each independently selected from hydrogen, -NH₂, -OH, halogen, carbonyl, C₁-C₄ alkyl, C₁-C₄ alkoxy, C₃-C₈ cycloalkyl and C₁-C₄ alkylamino;
R₈ and R₉ are each independently selected from -NH₂, -OH, -CN, -COOH, halogen, C₁-C₄ alkyl, C₁-C₄ alkoxy, and C₁-C₄ alkylamino; wherein the C₁-C₄ alkyl is optionally substituted by one to three substituents selected from -NH₂, -OH, and -CN;
alternatively, R₈ and R₉ form a 3- to 7-membered saturated or unsaturated ring together with the carbon atom connected thereto, and the 3- to 7-membered saturated or unsaturated ring optionally contains one to three heteroatoms or groups independently selected from N, O, C(=O), and S(=O)ₘ, wherein m=1 or 2; the saturated or unsaturated ring is optionally substituted by one to three substituents selected from -NH₂, -OH, -CN, halogen, C₁-C₄ alkyl, C₁-C₄ alkoxy, and C₁-C₄ alkylamino;
Y is CR₁₂ or N;
R₁₂ is selected from hydrogen, -CN and halogen;
Z₁ is N or CR₁₅;
R₁₅ is selected from hydrogen, -CN, halogen, C₁-C₄ alkyl, C₃-C₈ cycloalkyl, 3- to 10-membered heterocyclyl, C₆-C₁₀ aryl and 5- to 10-membered heteroaryl; the C₁-C₄ alkyl, C₃-C₈ cycloalkyl, 3- to 10-membered heterocyclyl, C₆-C₁₀ aryl, 5- to 10-membered heteroaryl are optionally further substituted by one to three substituents independently selected from -NH₂, -OH, -CN, halogen, C₁-C₄ alkyl, C₃-C₆ cycloalkyl and C₁-C₄ alkoxy.

4. The compound, or a pharmaceutically acceptable salt, enantiomer, diastereomer, atropisomer, racemate, polymorph, solvate or isotopically labeled compound thereof according to claim 1, wherein the compound represented by formula I is selected from the compounds represented by formula Ic: 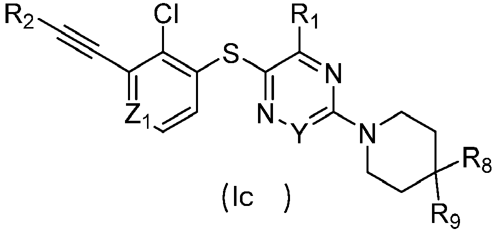 wherein
R₁ is selected from hydrogen and -NH₂;
R₂ is selected from hydrogen, C₁-C₁₀ alkyl, C₃-C₈ cycloalkyl, 3- to 10-membered heterocyclyl, C₆-C₁₀ aryl, and 5- to 10-membered heteroaryl; the C₁-C₁₀ alkyl, C₃-C₈ cycloalkyl, 3- to 10-membered heterocycloalkyl, C₆-C₁₀ aryl, and 5- to 10-membered heteroaryl are optionally further substituted by one to five substituents selected from -NR₁₀R₁₁, -OR₁₀, -CN, halogen, C₁-C₃ alkyl, C₃-C₆ cycloalkyl, -C(=O)R₁₀, -C(=O)OR₁₀, -C(=O)NR₁₀R₁₁, -S(=O)₂R₁₀, -S(=O)₂NR₁₀R₁₁, and -NR₁₀C(=O)R₁₁; wherein, R₁₀ and R₁₁ are independently selected from hydrogen and C₁-C₄ alkyl;
R₈ and R₉ are each independently selected from -NH₂, -OH, -CN, -COOH, halogen, C₁-C₄ alkyl, C₁-C₄ alkoxy, and C₁-C₄ alkylamino; wherein the C₁-C₄ alkyl is optionally substituted by one to three substituents selected from -NH₂ and -OH;
alternatively, R₈ and R₉ form a 3- to 7-membered saturated or unsaturated ring together with the carbon atom connected thereto, and, the 3- to 7-membered saturated or unsaturated ring optionally contains one to three heteroatoms or groups independently selected from N, O, C(=O), and S(=O)ₘ, wherein m=1 or 2; the saturated or unsaturated ring is optionally substituted by one to three substituents selected from -NH₂, -OH, C₁-C₄ alkyl, C₁-C₄ alkoxy, C₁-C₄ alkylamino and halogen;
Y is CH or N;
Z₁ is N or CR₁₅;
R₁₅ is selected from hydrogen, -CN, C₁-C₄ alkyl, C₃-C₈ cycloalkyl and 3- to 10-membered heterocyclyl.

5. The compound, or a pharmaceutically acceptable salt, enantiomer, diastereomer, atropisomer, racemate, polymorph, solvate or isotopically labeled compound thereof according to claim 1, wherein the compound represented by formula I is selected from the compounds represented by formula Id: 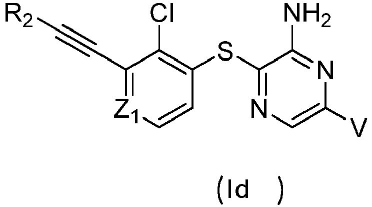 wherein
R₂ is selected from hydrogen, C₁-C₁₀ alkyl, C₃-C₈ cycloalkyl, 3- to 10-membered heterocyclyl, C₆-C₁₀ aryl, and 5- to 10-membered heteroaryl; the C₁-C₁₀ alkyl, C₃-C₈ cycloalkyl, 3- to 10-membered heterocycloalkyl, C₆-C₁₀ aryl, and 5- to 10-membered heteroaryl are optionally further substituted by one to two substituents selected from -NR₁₀R₁₁, -OR₁₀, -CN, halogen, C₁-C₃ alkyl, C₃-C₆ cycloalkyl, -C(=O)R₁₀, -C(=O)OR₁₀, -C(=O)NR₁₀R₁₁, -S(=O)₂R₁₀, -S(=O)₂NR₁₀R₁₁, and -NR₁₀C(=O)R₁₁; wherein, R₁₀ and R₁₁ are independently selected from hydrogen and C₁-C₄ alkyl;
Z₁ is N or CR₁₅;
R₁₅ is selected from hydrogen, -CN, C₁-C₄ alkyl, C₃-C₈ cycloalkyl and 3- to 10-membered heterocyclyl;

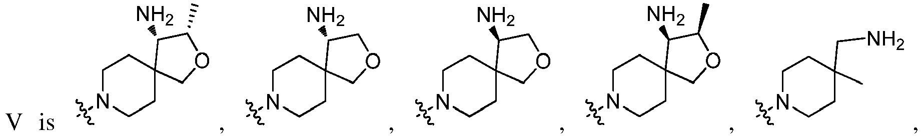

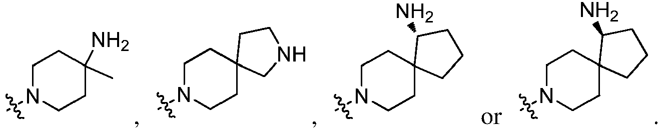

6. The compound, or a pharmaceutically acceptable salt, enantiomer, diastereomer, atropisomer, racemate, polymorph, solvate or isotopically labeled compound thereof according to claim 1, wherein the compound represented by formula I is selected from the compounds represented by formula Ie: 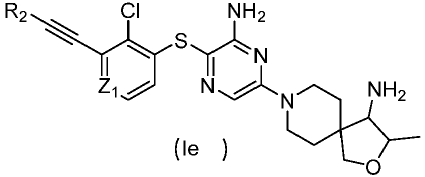 wherein
R₂ is selected from C₁-C₁₀ alkyl, C₃-C₈ cycloalkyl, 3- to 10-membered heterocyclyl, C₆-C₁₀ aryl and 5- to 10-membered heteroaryl; the C₁-C₁₀ alkyl, C₃-C₈ cycloalkyl, 3- to 10-membered heterocyclyl, C₆-C₁₀ aryl, 5- to 10-membered heteroaryl are optionally further substituted by one to two substituents independently selected from halogen, C₁-C₃ alkyl and C₃-C₆ cycloalkyl;
Z₁ is N or CR₁₅;
R₁₅ is selected from hydrogen, C₁-C₄ alkyl and C₃-C₈ cycloalkyl.

7. The compound, or a pharmaceutically acceptable salt, enantiomer, diastereomer, atropisomer, racemate, polymorph, solvate or isotopically labeled compound thereof according to claim 1, wherein the compound represented by formula I is selected from:

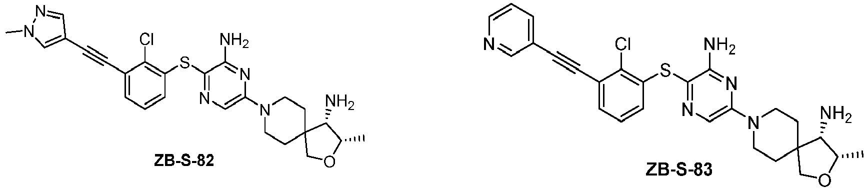

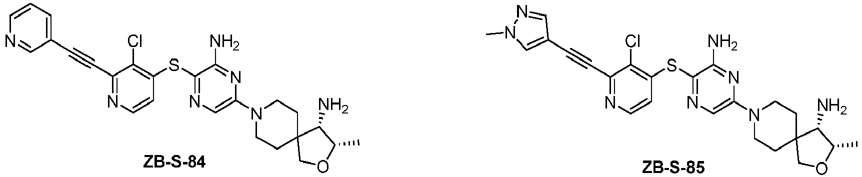

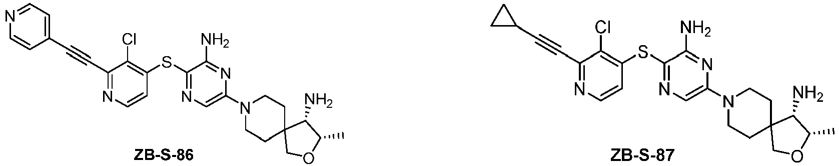

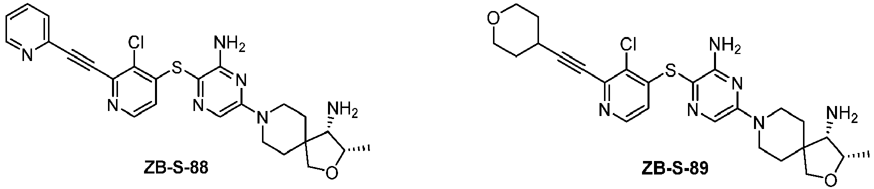

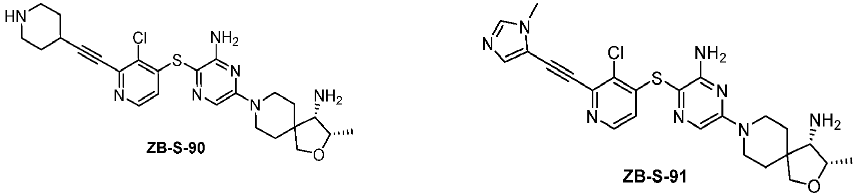

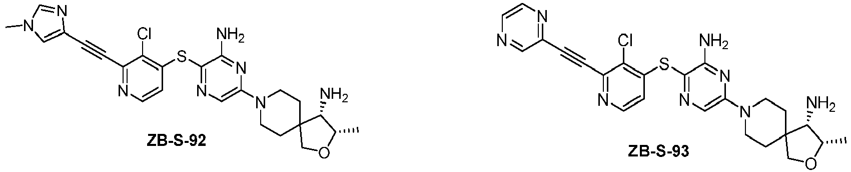

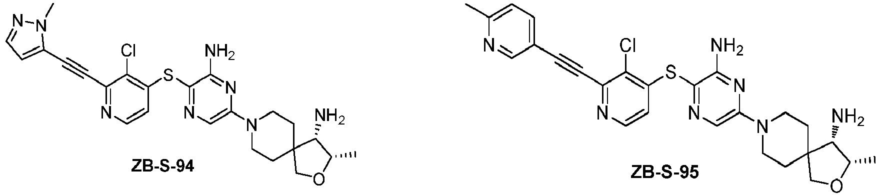

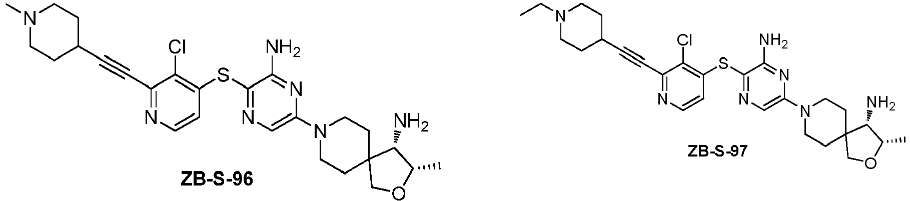

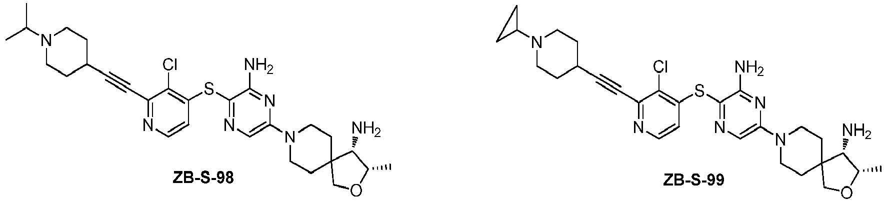

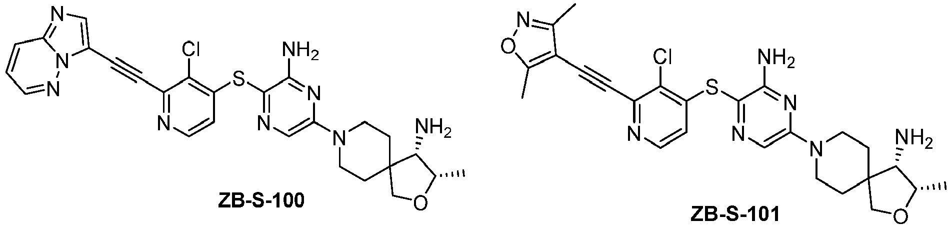

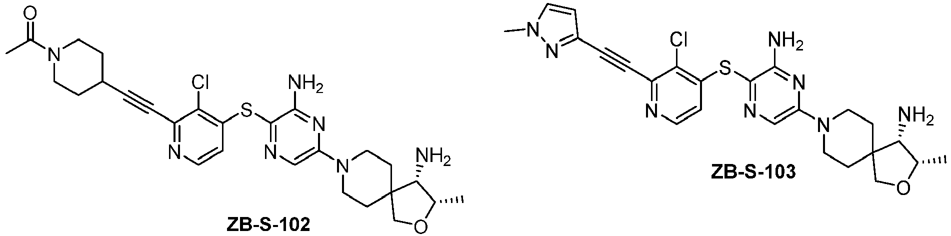

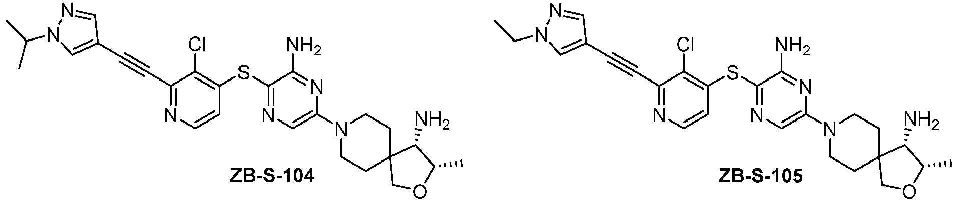

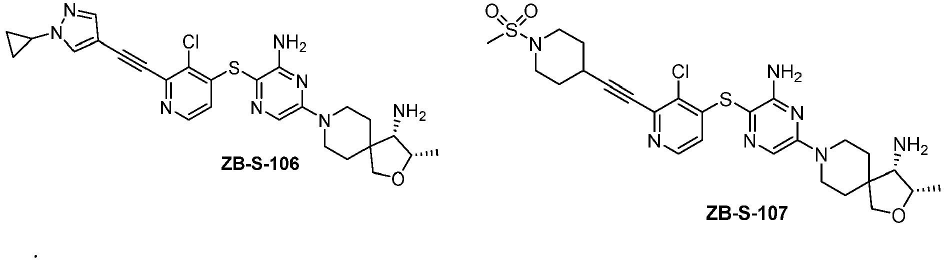

8. A pharmaceutical composition, comprising a therapeutically effective amount of one or more selected from the compound, the pharmaceutically acceptable salt, enantiomer, diastereomer, atropisomer, racemate, polymorph, solvate and isotopically labeled compound thereof according to any one of claims 1 to 7, and a pharmaceutically acceptable carrier.

9. Use of the compound, or the pharmaceutically acceptable salt, enantiomer, diastereomer, atropisomer, racemate, polymorph, solvate or isotopically labeled compound thereof according to any one of claims 1 to 7, or the pharmaceutical composition according to claim 8 in preparing a medicament for treating a disease or disorder or condition mediated by SHP2.

10. The use according to claim 9, wherein, the disease or disorder or condition is selected from: Noonan syndrome, leopard skin syndrome, leukemia, neuroblastoma, liver cancer, neuroblastoma, head and neck squamous cell carcinoma, melanoma, breast cancer, esophageal cancer, head and neck tumor, gastric cancer, lung cancer, colon cancer, anaplastic large cell lymphoma, glioblastoma, and reproductive system tumors.
